# EUROPEAN PATENT APPLICATION

(11) **EP 4 640 167 A1**
(43) Date of publication of application: **29.10.2025**
(21) Application number: 23910978.8
(22) Date of filing: 29.12.2023
(51) Int. Cl.: A61B 17/068, A61B 17/072, A61B 17/115

(54) **DRIVING STRUCTURE USED FOR SURGICAL SUTURING INSTRUMENT AND SURGICAL SUTURING INSTRUMENT**

(30) Priority: 30.12.2022 WO PCT/CN2022/144341; 26.06.2023 CN 202310765042; 26.06.2023 CN 202310765128
(71) Applicant: Wuhan United Imaging Surgical Co., Ltd., Wuhan, Hubei 430073 (CN)
(72) Inventor: WANG, Zhe, Wuhan, Hubei 430073 (CN)
(74) Representative: Wang, Bo
(86) International application number: PCT/CN2023/143474
(87) International publication number: WO 2024/141065

(57) **Abstract**

This embodiment of the specification provides a driving structure for surgical suture instruments and surgical suture instruments. The drive structure is arranged in a handle housing of the handle section of the surgical suture instrument, and the drive structure includes: a rack that can be linearly arranged in the handle housing; A trigger set on the handle housing can be turned; Drive assembly, the drive assembly includes a drive claw connected to the trigger movement; Mode switching mechanism, including the operating component and the latch connected to the rack movement; The operating component is connected to the drive assembly; The operation component controls whether the drive component fits with the latch, thereby controlling the drive claw to switch between the first drive mode and the second drive mode.

## Description

### CROSS-REFERENCE TO RELATED APPLICATION

This application claims priority for the PCT application with application number PCT/CN2022/144341 filed on December 30, 2022, and priority for the Chinese application with application number 202310765042.3 filed on June 26, 2023. Priority of the Chinese application with application number 202310765128.6, filed on June 26, 2023, is combined herein by reference.

### FECHNICAL FIELD

This specification pertains to the technical field of medical devices, particularly to a driving structure for surgical suturing instruments and surgical suturing instruments.

### BACKGROUNG

Compared with traditional open surgery, minimally invasive surgery causes less damage and trauma to tissues, less bleeding, and patients recover faster after surgery. As a result, minimally invasive surgery has become the constant pursuit of doctors to replace traditional open surgery. During the operation, suturing is often needed to restore the continuity of tissues and organs. In medicine, suturing devices such as staplers can be used as alternatives to traditional manual suturing, with which the lesion area can be sutured while being cut. The suturing apparatus can usually be used for tissue compression or for hair suturing. For surgical suturing instruments, how to switch modes conveniently (such as between tissue compression mode and hair suturing mode) is a technical problem that needs to be solved urgently in this field.

### SUMMARY

One or more embodiments of the present Specification provide a drive structure for a surgical suturing instrument, which is arranged in a handle housing of the handle part of the surgical suturing instrument, and the drive structure includes: a rack that can be arranged in a linear motion on the handle housing; A trigger provided on the handle housing can be rotated; Drive assembly, wherein the drive assembly includes a drive claw connected to the trigger movement; Mode switching mechanism, including an operating component and a latch connected to the rack activity; The operating component is connected to the driving component; The operation component controls whether the drive component fits with the latch, thereby controlling the switch of the drive claw between the first drive mode and the second drive mode.

One or more embodiments of the present Specification provide a surgical suture instrument, including a handle unit, an end actuator and a drive structure. The operation of the handle unit regulates the working mode of the end actuator through the drive structure, which includes the drive structure mentioned above.

### BRIEF DESCRIPTION OF THE DRAWINGS

This specification will be further illustrated in the form of exemplary embodiments, which will be described in detail by the attached figures. These embodiments are not restrictive. In these embodiments, the same numbering represents the same structure wherein:
Figure 1 is a structural schematic diagram of the driving structure for surgical suture instruments as shown in some embodiments of this specification;
Figure 2 is a schematic diagram of the structure of the drive structure for surgical suturing instruments as shown in some embodiments of this specification;
Figure 3 is a partial structural diagram of the drive structure for surgical suturing instruments as shown in some embodiments of this specification;
Figure 4A is a partial structural diagram of the drive structure for surgical suturing instruments as shown in some embodiments of this specification;
Figure 4B is an enlarged diagram of point A in Figure 3A as shown in some embodiments of the present Specification;
Figure 5 is a schematic diagram of another structure of the latch as shown in some embodiments of this specification;
Figure 6 is a schematic diagram of the explosion of the drive claw as shown in some embodiments of this specification;
Figure 7 is a schematic diagram of the first operating device as shown according to some embodiments of this specification;
Figure 8A is a schematic diagram of the operation mode of the first operating device according to some embodiments of this specification;
Figure 8B is a schematic diagram of the operation mode of the first operating device as shown in some embodiments of this specification;
Figure 8C is a schematic diagram of the operation mode of the first operating device as shown in some embodiments of this specification;
Figure 9A is a schematic diagram of the structure of the button hole as shown in some embodiments of this specification;
Figure 9B is a schematic diagram of the structure of the button hole as shown in some embodiments of this specification;
Figure 10 is a schematic diagram of the initial mode as shown according to some embodiments of this specification;
Figure 11 is a schematic diagram of the first pattern as shown according to some embodiments of this specification;
Figure 12 is a schematic diagram of the first pattern as shown according to some embodiments of this specification;
Figure 13 is a schematic diagram of the first mode as shown in some embodiments of the present specification;
Figure 14 is a schematic diagram of mode switching according to some embodiments of this specification;
Figure 15 is a schematic diagram of the second mode as shown according to some embodiments of this specification;
Figure 16 is a schematic diagram of the second pattern as shown according to some embodiments of the present Specification;
Figure 17 is a schematic diagram of the reset to the initial mode as shown in some embodiments of this specification;
Figure 18 is a schematic diagram of resetting to initial mode as shown in some embodiments of this specification;
Figure 19 is a schematic diagram of resetting to initial mode as shown in some embodiments of this specification;
Figure 20 is a structural diagram of the drive structure as shown in some other embodiments of this specification;
Figure 21 is a schematic diagram of the second operating piece as shown in some other embodiments of this specification;
Figure 22 is a schematic diagram of the installation of the second operating piece as shown in some other embodiments of this specification;
Figure 23 is a schematic diagram of the initial mode as shown according to some other embodiments of this specification;
Figure 24 is a schematic diagram of the first pattern as shown according to some other embodiments of this specification;
Figure 25 is a schematic diagram of the first pattern as shown according to some other embodiments of this specification;
Figure 26 is a schematic diagram of the first pattern as shown according to some other embodiments of this specification;
Figure 27 is a schematic diagram of the mode switching as shown in some other embodiments of this specification;
Figure 28 is a schematic diagram of the second mode as shown according to some other embodiments of this specification;
Figure 29 is a schematic diagram of the second mode as shown in some other embodiments of the present specification;
Figure 30 is a schematic diagram of the reset to the initial mode as shown in some other embodiments of this Specification;
Figure 31 is a schematic diagram of resetting to initial mode as shown in some other embodiments of this specification;
Figure 32 is a schematic diagram of resetting to initial mode as shown in some other embodiments of this specification;
Figure 33 is a structural diagram of the drive structure as shown in some other embodiments of this specification;
Figure 34 is a partial structural diagram of the drive structure as shown in some other embodiments of the present Specification;
Figure 35A is a schematic diagram of the structure of the latch as shown in some other embodiments of this specification;
Figure 35B is an explosion diagram of the latch as shown in some other embodiments of the present Specification;
Figure 36A is a schematic diagram of the structure of the drive claw as shown in some other embodiments of this specification;
Figure 36B is an explosion diagram of the drive claw as shown in some other embodiments of this specification;
Figure 37 is a schematic diagram of the operating end of the operating component as shown in some other embodiments of this specification;
Figure 38A is a schematic diagram of the structure of the operating device according to some other embodiments of the present Specification;
Figure 38B is a schematic diagram of the structure of the operating part according to some other embodiments of the present Specification;
Figure 38C is a schematic diagram of the structure of the operating part according to some other embodiments of the present Specification;
Figure 39 is an explosion diagram of the operating device as shown in some other embodiments of the present Specification;
Figure 40 is a schematic diagram of the structure of the latch and rack as shown in some other embodiments of this specification;
Figure 41 is a schematic diagram of the initial mode as shown in some other embodiments of the present specification;
Figure 42 is a schematic diagram of the first pattern as shown according to some other embodiments of this specification;
Figure 43 is a schematic diagram of the first pattern as shown according to some other embodiments of this specification;
Figure 44 is a schematic diagram of the first pattern as shown according to some other embodiments of this specification;
Figure 45 is a schematic diagram of the first pattern as shown according to some other embodiments of this specification;
Figure 46 is a schematic diagram of the mode switching as shown in some other embodiments of this specification;
Figure 47 is a schematic diagram of the mode switching as shown according to some other embodiments of this specification;
Figure 48 is a schematic diagram of the mode switching as shown in some other embodiments of this specification;
Figure 49 is a schematic diagram of the second mode as shown according to some other embodiments of this specification;
Figure 50 is a schematic diagram of the second pattern as shown according to some other embodiments of the present Specification;
Figure 51 is a schematic diagram of the second pattern as shown according to some other embodiments of the present Specification;
Figure 52 is a schematic diagram of the second pattern as shown according to some other embodiments of the present Specification;
Figure 53 is a schematic diagram of the tissue release operation as shown in some other embodiments of this specification.

### DETAILED DESCRIPTION

To illustrate the technical solution of the embodiments of this specification more clearly, a brief introduction to the accompanying drawings required in the description of the embodiments is provided below. It is obvious that the drawings described below are only examples or embodiments of this specification, which can be used by those skilled in the art to apply this specification to other similar scenarios without creative effort. Unless it is obvious from the context of the language or otherwise stated, the same markings in the figures represent the same structure or operation.

A suturing device/stapler is a medical suturing instrument used as a substitute for traditional manual suturing. Its main working principle is to separate or anastomose the tissue with a sewing pin, and the lesion area can be sutured while cutting. Compared with manual suturing, because the nails are neatly arranged and spaced at equal intervals, the tightness of the suturing is controllable, avoiding manual suturing that is too sparse or too dense and binding that is too tight or too loose, ensuring good healing of the tissue. The main components of the sewing instrument may include a nail drill, nail box, nail compartment, nail drive, handle, positioning needle, etc. To remove excess tissue, various tools such as ring knives, push knives, etc. can be equipped. Compared with manual suturing, mechanical suturing and anastomosis are simple and quick to operate, greatly reducing the operation time; Accurate, firm and reliable, maintaining good blood supply, better guaranteed tissue healing, effectively preventing leakage, significantly reducing the incidence of anastomotic leakage; Mechanical suturing makes it easier to suture and anastomoses that are difficult for manual operation when the field is small and the area is deep. Changing manual open suturing or anastomosis to closed suturing and anastomosis reduces the chance of contamination of the surgical field during digestive tract reconstruction and bronchial stump closure; Cross and repeat suturing can be performed to avoid blood supply and tissue necrosis; The application of various endoscopic suturing devices has made endoscopic and laparoscopic surgery possible.

Traditional surgical techniques include cutting, separation, ligation, hemostasis and suturing, ultimately achieving the resection of organ lesions and the reconstruction of organs. Mechanical suturing can replace conventional surgical techniques and can achieve the resection and reconstruction of diseased organs through operations such as amputation, suturing and anastomosis. Dissection involves suturing organs at a certain distance from the lesion using a suturing device, including solid organs, cavity organs, blood vessels, etc., and then dissecting and removing the lesion, or using a linear cutting suturing device to complete the suturing and dissection in one go. Examples include thyroid lobectomy, lobectomy, pulmonary wedge resection, colonic dissection, gastric dissection, etc. Suturing involves joining the tissue to be sutured and sewing it with a linear suturing machine, such as making longitudinal and transverse cuts at the pylorus to complete pyloroplasty. Anastomosis, using a circular stapler, can conveniently perform end-to-end anastomosis and end-side anastomosis of cavity organs such as the esophagus, stomach, small intestine, and colon. Gastrointestinal side anastomosis is performed using a cutting and suture device. For example, rectocolonic end-to-end anastomosis, esophagogastric end-side anastomosis, and gastrojejunoside-side anastomosis, etc. For different operations such as amputation, suturing and anastomosis, the suturing device can correspond to different operation modes. For example, a suturing device can have a squeezing mode, that is, the ends of the suturing device can perform a squeezing operation, for example, the jaws at the ends of the suturing device can be closed, thereby clamping and further squeezing the tissue between the jaws; The suturing device may have a firing mode, which includes two steps: pushing nail shaping and pushing knife cutting, to complete the suturing while performing the cutting.

To meet clinical needs, some embodiments of this specification provide a surgical suturing instrument which includes a handle with a movable trigger, a drive structure and an end actuator. The drive structure can be set inside the handle, and the end actuator is connected to the drive structure through a connecting component. The end actuator includes a tool assembly with clamping, cutting and suturing functions, for example, a jaws, a cutter and a pinning device, for performing various working modes such as tissue compression, cutting and suturing. The drive structure can be used to select and switch the working mode of the surgical suture instrument, and drive the end actuator to perform the corresponding operation in different working modes. For example, it is used to switch between pressing mode and firing mode, and to control the surgical suture instrument to perform the operation corresponding to the selected mode.

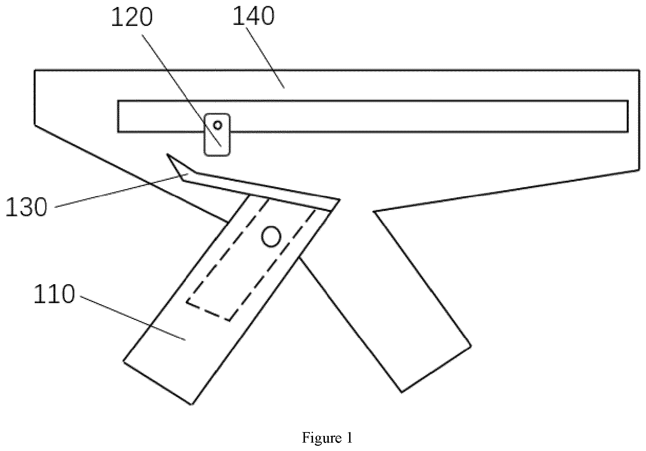

Figure 1 is a structural diagram of the drive structure for surgical suturing instruments as shown in some embodiments of this specification.

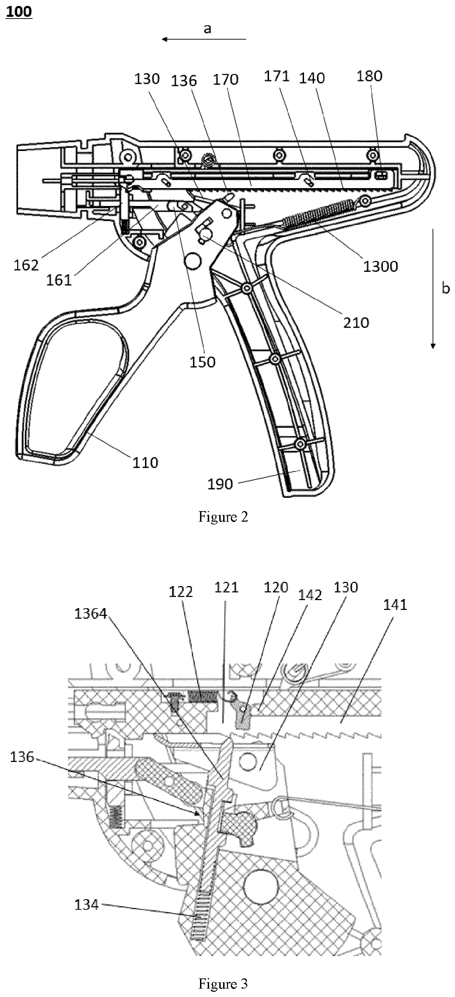

As shown in Figure 1, the embodiments of this specification also provide a drive structure for a surgical suturing instrument, which is arranged in a handle housing of the handle part of the surgical suturing instrument. The drive structure includes a rack 140 that can be arranged in a linear motion on the handle housing, a trigger 110 that can be rotated on the handle housing, a drive assembly and a mode switching mechanism. The drive assembly includes a drive claw 130 which is movably connected to the trigger, and the mode switching mechanism includes an operating component and a latch which is movably connected to the rack 140; The operation assembly is connected to the drive assembly; The operation component controls whether the drive component fits with the latch 120, thereby controlling the drive claw 130 to switch between the first drive mode and the second drive mode. Among them, the coordination of the drive assembly with the latch 120 can be the coordination of the drive claw 130 with the latch or the coordination of other structures of the drive assembly (limit part 1264 as shown in Figure 2) with the latch 120.

Some embodiments of this specification achieve the switching of the driving claw between the first driving mode and the second driving mode by operating the component, thereby realizing the switching of the working mode of the surgical suture instrument. The operation is very convenient, smooth and does not affect the operator's grip and other operations of the surgical suturing instrument.

Figure 2 is a schematic diagram of the structure of the drive structure 100 for surgical suturing instruments as shown in some embodiments of this specification. Figure 3 is a partial structural diagram of the drive structure 100 for surgical suturing instruments as shown in some embodiments of this specification.

In some embodiments, as shown in Figures 2 and 3, the drive structure 100 for the surgical suturing instrument may include a trigger 110, a rack 140, a drive claw 130 and a mode switching mechanism. Among them, the rack 140 can move in a straight line, the trigger 110 can rotate, the drive claw 130 can be movably connected to the trigger 110, and the mode switching mechanism can be set on the trigger 110. In some embodiments, the drive claw 130 is used in conjunction with the teeth of the rack 140 for power transmission. In some embodiments, the drive claw 130 can be movably connected to the trigger 110, and the operator can drive the power to the drive claw 130 by pulling the trigger 110, which then drives the power to the rack 140, thereby driving the rack 140 to move. In some embodiments, rack 140 refers to the structure where the teeth are distributed over the strip body. Rack 140 can move in a straight line. In some embodiments, rack 140 can be configured to be able to move forward in the direction of arrow a as shown in Figure 1.

The function of the mode switching mechanism is to control the drive claw 130 to switch between the first drive mode and the second drive mode. In some embodiments, in the first drive mode, the surgical suturing instrument corresponds to the execution of the first mode (such as the squeezing mode). In some embodiments, in the second drive mode, the surgical suturing instrument can perform the second mode (such as the firing mode). In some embodiments, the operating component of the mode switching structure can control the drive claw 130 to switch between the first drive mode and the second drive mode by controlling whether the drive component cooperates with the latch 120. The operation component and the drive component can have multiple structural forms, which can be further described below. It should be noted that depending on the structure of the operating and driving components, the latch works differently and the way the latch fits with the driving components will vary.

In some embodiments, as shown in Figures 2-19, the mode switching mechanism may include a latch 120 that can be movably connected to the rack 140, the operating component may include an operable switching slider 136, and the driving component may include a limit part 1364 that is fixedly connected to the switching slider 136. In some embodiments, the limit part 1364 can move the drive claw 130, for example, the drive claw 130 May be provided with an opening for the limit part 1364 to pass through, and the limit part 1364 May pass through the drive claw 130 from bottom to top (in the opposite direction of b in Figure 2), with the opening size on the drive claw 130 being larger than the size of the limit part 1364. In some embodiments, the switching slider 136 can drive the limit unit 1364 to move between the first position and the second position, allowing the drive claw 130 to switch between the first drive mode and the second drive mode. In some embodiments, when the limit part 1364 switches from the first position to the second position, it causes the limit part 1364 to switch from being confined within the preset travel by the latch 120 to the unrestricted state, thereby enabling the drive claw 130 to switch from the first drive mode to the second drive mode. In some embodiments, in the first drive mode, the limit unit 1364 is confined within the preset travel by the latch 120. In some embodiments, the range of the preset stroke is related to the range to which the limit part 1364 is restricted by the latch 120, for example, the limit part 1364 is restricted by the latch 120 in the first limit slot 121 of the rack 140, and the distance that the limit part 1364 can move within the first limit slot 121 is positively correlated with the preset stroke. In some embodiments, the limit unit 1364 is confined in the first limit slot 121, and the trigger 110 can drive the limit unit 1364 to move within a certain range in the first limit slot 121, thereby enabling the drive claw 130 to drive the rack 140 forward or backward within a certain stroke.

The following will provide a detailed description of the components of the drive structure 100, and it should be noted that the following embodiments are only for illustrative illustration of the implementation of the drive structure 100 and its components.

Trigger 110 is a component for the operator to pull, and the operator can transmit the force applied by the operator to other components connected to trigger 110 by pulling the trigger 110. In some embodiments, the operation of the trigger 110 can also drive the movement of the rack 140 in different modes to achieve related operations such as pressing or sewing. In the example, in the second mode, the trigger 110 can perform cutting and sewing by moving the rack 140 and the tool assembly attached to the rack 140 (such as a cutter, pinning device, etc.) forward. In some embodiments, the trigger 110 is rotatable so that the operator can move it. In some embodiments, the trigger 110 can be movably connected to other components, for example, the trigger 110 can be pivotly connected to the drive claw 130 to deliver power. In some embodiments, the trigger 110 can be movably connected to the handle housing 190, which can be used to install and carry the various functional components of the drive structure 100, and the trigger 110 can rotate relative to the handle housing 190. In some embodiments, the range of rotation of the trigger 110 can be limited to a certain Angle, for example, the range of rotation of the trigger 110 can be 30°-80°, and the range of rotation Angle can be set as needed.

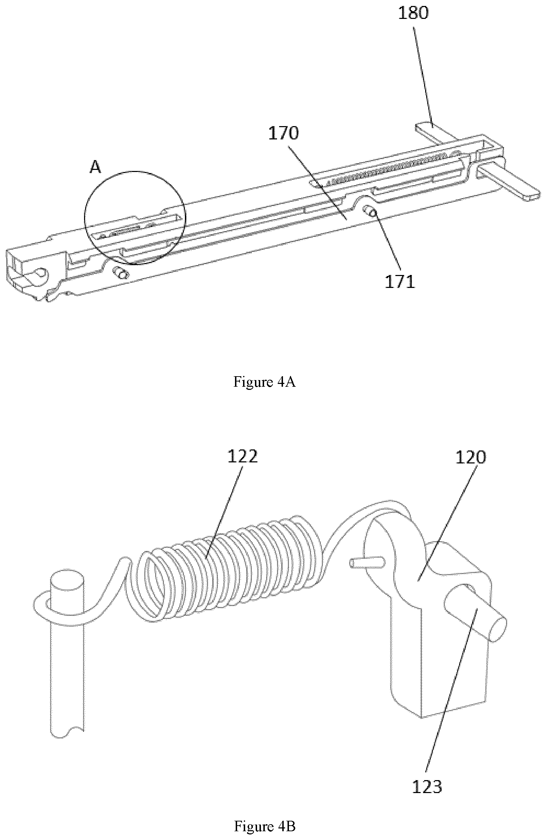

Latch 120 can be used to limit the relative positions of two or more objects. In some embodiments, the latch 120 can be movably connected with the rack 140, and the latch 120 can be coordinated with the rack 140 to limit the movement of the limit part 1364. For example, the latch 120 can be used in conjunction with the first limit slot 121 of the rack 140 to confine the limit part 1364 within the first limit slot 121; The limit part 1364 can be detached from the first limit slot 121, thereby freeing the first limit slot 121 from restricting the movement of the limit part 1364. For details of the connection between the latch 120 and the rack 140, see Figures 4A and 4B and their related descriptions.

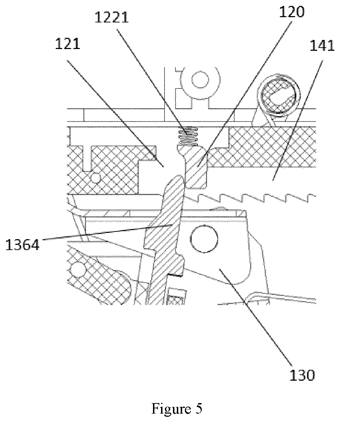

Figure 4A is a partial structural diagram of the drive structure 100 for surgical suturing instruments as shown in some embodiments of this specification. Figure 4B is an enlarged diagram of point A in Figure 4A as shown according to some embodiments of this specification. Figure 5 is a schematic diagram of another structure of latch 120 as shown in some embodiments of this specification.

In some embodiments, the latch 120 can take on various shapes, for example, triangular, cuboid, cylindrical, etc. In some embodiments, as shown in Figure 4A, latch 120 May be set on rack 140, rack 140 May have multiple teeth, latch 120 May be set at the distal end of multiple teeth on rack 140, the distal end may be relative to the proximal end, and for surgical suturing instruments, the end closer to the operator may be considered as the proximal end. The end away from the operator is the distal end (the direction corresponding to the distal end can be seen as direction a in Figure 2). In some embodiments, the rack 140 (see b direction in Figure 2) is provided with a first limit slot 121, and the latch 120 can be pivot set near the end of the first limit slot 121 of the rack 140. When the limit part 1364 is located in the first limit slot 121, the distal side wall of the first limit slot 121 can restrict the forward movement of the limit part 1364. The distal side wall of the latch 120 can restrict the backward movement of the limit unit 1364, with the distal side wall of the first limit slot 121 facing the distal side wall of the latch 120. Among them, the forward movement can correspond to the movement in the forward direction of the rack 140 (see a direction in Figure 2). In some embodiments, the latch 120 May be set in the first limit slot 121 with a clamping connection (movable ground), a pivot connection, or any other viable connection method so that the latch 120 should be able to present a state of being fully or partially placed in the first limit slot 121, or a state of protruding downward relative to the rack 140.

In some embodiments, the latch 120 May be rotatably connected to the rack 140, as shown in Figure 4B, the latch 120 and the rack 140 are rotatably connected by the rotating shaft 123 so that the latch 120 can be rotated from being fully or partially placed in the first limit slot 121 to protruding downward relative to the rack 140, or by protruding downward relative to the rack 140, Rotate to be fully inserted into the first limit slot 121. In some embodiments, the limit part 1364 is able to drive the latch 120 to rotate, causing the latch 120 to rotate from a position downward relative to the rack 140 within the first limit slot 121 until the limit part 1364 disengages from the latch 120 and the latch 120 returns to a position downward relative to the rack 140. In some embodiments, in the case where the latch 120 is downward relative to the rack 140, the movement of the limit unit 1364 would be restricted if the limit unit 1364 were in the first limit slot 121.

In some embodiments, as shown in Figure 3, the rack 140 May have a stop unit 142, which may be located within the first limit slot 121, and the stop unit 142 May be set at the rear end of the first limit slot 121. In some embodiments, the stop 142 May be in contact with the latch 120 to limit the Angle at which the latch 120 rotates towards the proximal end of the rack 140 (counterclockwise as shown in Figure 3). For example, the latch 120 can rotate counterclockwise around the rotating shaft 123 to the position shown in Figure 3, which is blocked by the latch 142 and cannot continue to rotate. In some embodiments, by setting the stop 142, the rotation Angle of the latch 120 can be controlled, thereby limiting the limit part 1364 through the latch 120 to control the drive mode in which the drive claw 130 is located.

In some embodiments, the limit part 1364 can drive the latch 120 to rotate away from the stop part 142 from the initial position (see the clockwise direction in Figure 3), and after the limit part 1364 passes the limit position, the latch 120 resets to the initial position, and the latch 120 contacts the stop part 142 at the initial position. This restricts the backward movement of the limit part 1364, which also limits the movement of the drive claw 130 when the drive claw 130 is in the first drive mode. In some implementations, the initial position can be downward relative to the rack 140, and in the initial position, the latch 120 can only rotate around the rotating shaft 123 in a single direction away from the stop 142; The limit position may refer to the critical position where the limit part 1364 is about to separate from the latch 120 and no longer continue to contact. In some embodiments, the limit unit 1364 can be selectively operated by the operator to make contact with the latch 120, driving the latch 120 to move in a single direction starting from the initial position until the limit unit 1364 is restricted by the first limit slot 121 and the latch 120, thereby achieving mode switching.

In some embodiments, the latch 120 May include an elastic resetting element, and the latch 120 is connected to the rack 140 through the elastic resetting element. In some embodiments, the elastic resetting element may apply force to the latch 120 such that the latch 120 is pressed against the stopper 142, and when the latch 120 moves away from the stopper 142 under force, and when the force is less than the elastic force of the elastic resetting element or the force disappears, the latch 120 can easily rebound and thus return to its original position. In some embodiments, the latch 120 May not include an elastic reset element, and the latch 120 May be set to return to its original position by gravity.

In some embodiments, as shown in Figure 4B, the elastic reset element includes a tension spring element 122, with one end of the tension spring element 122 connected to the rack 140 and the other end of the tension spring element 122 connected to the latch 120. In some embodiments, one end of the tension spring 122 is attached to the rack 140 on the front side of the latch 120, and the other end of the tension spring 122 is attached to the side of the latch 120 close to the rotating shaft element 123. The elastic force exerted by the tension spring 122 on the latch 120 enables the latch 120 to be tightly in contact with the stopper 142 at its initial position and to be easily pulled back to its initial position after the latch 120 rotates away from the stopper 142 around the rotating shaft 123. The tension spring 122 is set in the same direction as the extension of the rack 140 (i.e., direction a in Figure 2), which is convenient for installation and saves space.

In some embodiments, the elastic resetting element may have other feasible Settings, for example, as shown in Figure 5, the elastic resetting element may include the spring element 1221. The latch 120 can be connected to the rack 140 through the spring element 1221, with the upper end of the spring element 1221 connected to the rack 140 and the lower end of the spring element 1221 connected to the latch 120. Under the elastic effect of spring element 1221, latch 120 can move up and down easily. By applying upward force through the limit part 1364 to compress spring element 1221, latch 120 can protrusion downward relative to rack 140 and rotate until it is fully or partially placed in the first limit slot 121. The limit part 1364 continues to move in the forward direction of the rack 140 until it separates from the latch 120 and stops in contact. Under the elastic force, the latch 120 can return to its original position, that is, protrude downward relative to the rack 140, thereby confining the limit part 1364 within the first limit slot 121. In some other embodiments, the elastic resetting element may include a torsion spring. For example only, the torsion spring can be set at 123 of the rotating shaft.

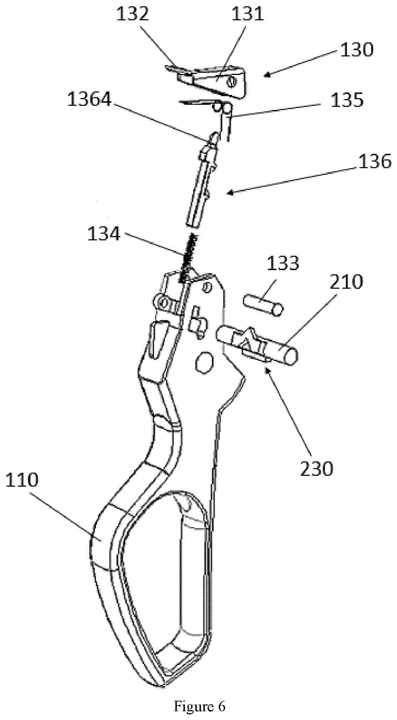

Figure 6 is a schematic diagram of the explosion of the driving claw 130 as shown in some embodiments of this specification.

In some embodiments, as shown in Figure 6, the drive claw 130 May include the main body part 131 and the end of the claw part 132, and the material of the end of the claw part 132 May be the same as or different from that of the main body part 131. In some embodiments, the material of the claw end 132 May be the same as that of the main body 131, for example, made of stainless steel. In some embodiments, the main body 131 can be fixedly connected to the end of the claws 132, the main body 131 can be PivotTable connected to the trigger 110, and the end of the claws 132 can be used to push the rack 140. In some embodiments, the end of the claw 132 can be inserted into the gap between the two teeth of the rack 140, thereby being able to exert a force on the rack 140 for functions such as pushing the rack 140 forward. In some embodiments, the tip 132 of the claw can be tilted to match the gap between the two teeth of the rack 140, so as to better fit into the gap between the two teeth of the rack 140 and avoid slippage. In some embodiments, the end 132 of the claw can be set in any shape that fits with the gap of rack 140 and is less likely to slip out.

In some embodiments, the drive claw 130 can be movably connected to the trigger 110. For example, the drive claw 130 can be pivotly connected to the trigger 110.

In some embodiments, the drive claw 130 can be set on the side of the trigger 110 close to the rack 140, and the trigger 110 can be rotatably connected to the drive claw 130 through the connecting shaft 133, which is provided with a torsion spring 135. When the trigger 110 is pulled, the trigger 110 can rotate around the center of the torsion spring 135 (i.e., the connecting shaft 133). The torsion spring 135 has the rotational force to return the trigger 110 to its original position, and thus the relative position of the trigger 110 and the drive claw 130 can be elastically restricted by the torsion spring 135. It should be noted that the torsion spring 135 is not a necessary structure and the trigger 110 can return to its original position in other ways, for example, by manual return, even without the torsion spring 135.

In some embodiments, the body part 131 is provided with an opening for the limit part 1364 to pass through. Since the limit part 1364 is threaded through the body part 131, therefore, when the movement of the limit part 1364 is restricted, the movement of the body part 131 is also restricted, that is, the movement of the drive claw 130 is restricted. In some embodiments, the extent to which the movement of the drive claw 130 is restricted can be adjusted by setting the size of the opening. For example, when the size of the opening is larger than that of the limit part 1364, after the movement of the limit part 1364 is restricted, the movement of the drive claw 130 within a certain range remains unrestricted until the drive claw 130 moves to the point where its main part 131 comes into contact with the limit part 1364 and the movement of the drive claw 130 begins to be restricted. Another example is that when the size of the opening is close to that of the limit 1364, the movement of the drive claw 130 is restricted when the movement of the limit 1364 is restricted.

In some embodiments, the drive claw 130 has the first drive mode and the second drive mode. For example only, in the first drive mode, the limit part 1364 can be locked into the first limit slot 121, and its forward movement is restricted by the first limit slot 121 and its backward movement is restricted by the latch 120 which is in the initial position, so that the drive claw 130 can only drive the rack 140 to move forward or backward within a certain range. In the second drive mode, when the limit part 1364 is outside the first limit slot 121, that is, when the limit part 1364 disengages from the first limit slot 121, the drive claw 130 is no longer restricted by the first limit slot 121 for its forward and backward movement. In some embodiments, the first drive mode of the drive claw 130 corresponds to the end actuator of the surgical suturing instrument performing the first mode (e.g., squeezing mode), and the second drive mode of the drive claw 130 corresponds to the end actuator of the surgical suturing instrument performing the second mode (e.g., firing mode). In some embodiments, the first drive mode and the second drive mode of the drive claw 130 can be switched manually or automatically. For example, the surgical suture instrument may include a first operating piece 210 that can be manually controlled by the operator by manually adjusting the state of the drive claw 130. This enables the end actuator of the surgical suture instrument to switch to either the first mode or the second mode. For more information on mode switching, see the relevant description below.

In some embodiments, the rack 140 is provided with rack steps and multiple teeth, and the drive claw 130 propels the rack 140 forward by cooperating with the rack steps or teeth.

In some embodiments, multiple teeth may be provided on the rack 140, and the coordination of the drive claw 130 with the teeth can push the rack 140 forward. In some embodiments, when the end actuator performs the second mode, the end 132 of the claw of the drive claw 130 holds against the teeth of the rack 140 and drives the drive claw 130 through the trigger 110, thereby pushing the rack 140 forward. In some embodiments, multiple teeth of the rack 140 can be straight or helical, etc. In some embodiments, when the teeth of rack 140 are helical, each helical tooth corresponds to the side of the forward direction of rack 140 as a slope, and each helical tooth on the side of the backward direction of rack 140 is perpendicular to the backward direction of rack 140; The inclined surface of the drive claw 130 with the inclined end of the inclined claw 132 can be matched with the inclined surface of the helical tooth, allowing the drive claw 130 to move easily towards the backward direction of the rack 140 on the surface of the helical tooth, and when the drive claw 130 moves towards the forward direction of the rack 140, The front end of the drive claw 130 can be in contact with the side of the helical tooth perpendicular to the backward direction of the rack 140, which ensures that the contact is less likely to loosen and can better exert force on the rack 140.

In some embodiments, a rack step may be provided on the rack 140, and the rack step may be a downward protrusion on the rack 140. The drive claw 130 can cooperate with the rack step, that is, the drive claw 130 pushes the rack 140 forward by pushing the protruding rack step. In some embodiments, during the execution of the first mode by the end actuator, when the limit part 1364 of the switching slider 136 is located in the first limit slot 121, the drive claw 130 is driven forward by the trigger 110, and the end 132 of the claw of the drive claw 130 touches the rack step of the rack 140. The end of the claw of the drive claw 130, 132, pushes the rack 140 forward by pushing the rack step, thereby enabling the end actuator of the surgical suture instrument to perform the clamping closure operation. In some embodiments, the rack step can be set at the distal end of the rack 140, and the latch 120 can be set between the teeth of the rack 140 and the rack step, making full use of the empty space on the rack 140 where teeth have not yet been distributed, resulting in a compact device structure. In some embodiments, a groove structure is provided on the rack step, and the anti-retreat slider 162 can be inserted into the groove structure so that the anti-retreat slider 162 can restrict the movement of the rack 140.

In some embodiments, a clamping device may be attached above the rack 140 to apply force downward to the rack 140. For example, the clamping device may be attached to the rack 140 through an elastic element, so that the clamping device can exert friction on the rack 140 to achieve a damping effect on the rack 140 and further prevent it from slipping. In some embodiments, the clamping device may be set on the handle housing 190.

In some embodiments, as shown in Figure 3, the rack 140 is provided with a avoidance slot 141 along the extension direction of the rack 140. The avoidance slot 141 is arranged on the side close to the drive claw 130 of the rack 140 and is located on the side near the end of the first limit slot 121 and is connected to the first limit slot 121. The avoidance slot 141 can accommodate the limit part 1364. In some embodiments, as the drive claw 130 drives the rack 140 forward, the limit part 1364 will move along with the drive claw 130, and since the limit part 1364 is inserted through the drive claw 130, it may come into contact with the teeth of the rack 140. If the end of the limit part 1364 touches the teeth of the rack 140, it will affect the movement of the rack, causing the movement of the rack 140 to be restricted or even impossible. Therefore, corresponding to the position of the limit part 1364, a avoidance slot 141 is set at the lower part of the rack 140. When the drive claw 130 drives the rack 140 forward (the limit part 1364 is not in the first limit slot 121), the limit part 1364 can be accommodated in the avoidance slot 141, thus not hindering or restricting the movement of the rack.

In some embodiments, the drive structure 100 May also include a pull spring 1300 (see Figure 2), with one end of the pull spring 1300 connected to the handle housing 190 and the other end connected to the trigger 110 for applying force to the trigger 110 in the opposite direction to the forward direction of the rack 140, so that the trigger 110 is not subjected to external force. The end of the trigger 110 connected to the spring 1300 has a tendency to move in the direction of retracting from the rack 140.

In some embodiments, as shown in Figure 2, the drive structure 100 May also include a forward slider 161 and a stop slider 162. The backstop slider 162 can be used to limit the movement of the rack 140. The forward slider 161 can remove the restriction imposed by the stop slider 162 on the movement of the rack 140 through the forward movement. The forward slider 161 can move in the forward direction. The backstop slider 162 can move up and down. In some embodiments, one end of the trigger 110 can be movably connected to the forward slider 161, and the stop slider 162 can be used to limit the movement of the rack 140, for example, the stop slider 162 can be movably set on the path in the forward direction of the rack 140. In some embodiments, the trigger 110 can move the stop slider 162 downward (see arrow b in Figure 2) by moving the forward slider 161 in the forward direction of the rack 140, thereby removing the restriction of the stop slider 162 on the movement of the rack 140.

In some embodiments, the backstop slider 162 can move up and down, the forward slider 161 can move in the forward direction of the rill 140, and the forward slider 161 is provided with a protruding protrusion that can contact the backstop slider 162. In some embodiments, as the forward slider 161 moves in the forward direction of the rack 140, the anti-retreat slider 162 is able to move downward under the push of the protrusions.

In some embodiments, the forward slider 161 can be inserted through the anti-retreat slider 162, and the anti-retreat slider 162 can play a certain guiding role for the forward slider 161 to ensure its forward direction. In some embodiments, the bottom of the anti-retreat slider 162 can be connected to the handle housing 190 by a spring member, and after the anti-retreat slider 162 loses the force of being pushed down and there is no obstruction above, the anti-retreat slider 162 can move upward to return to its initial position.

In some embodiments, when the end actuator performs the first mode, the stop slider 162 is in the lifted state at the starting position, thereby limiting the movement of the rack 140; Pull the trigger 110, and the trigger 110 drives the drive claw 130 forward until the limit part 1264 engages with the latch 120, and the limit part 1264 is in the first limit slot 121; Continuing to pull the trigger 110, the trigger 110 drives the forward slider 161 to move in the forward direction of the rack 140, which in turn drives the stop slider 162 to move downward, thereby lifting the restriction on the movement of the rack 140; At this point, continue to pull the trigger 110, and the drive claw 130 is pushed forward by the trigger 110. The end of the claw of the drive claw 130, 132, touches the rack step of the rack 140, thus enabling the rack 140 to move forward.

In some embodiments, the drive structure 100 May also include a connecting rod 150 (see Figure 2), which can be set between the forward slider 161 and the trigger 110, with one end of the connecting rod 150 rotatably connected to the forward slider 161 and the other end of the connecting rod 150 rotatably connected to the trigger 110 to exert the power of the operator to pull the trigger 110, The forward slider 161 is passed on. In some embodiments, the operator presses the trigger 110 through the connecting rod 150, which can push the forward slider 161 to move in the forward direction of the rack 140.

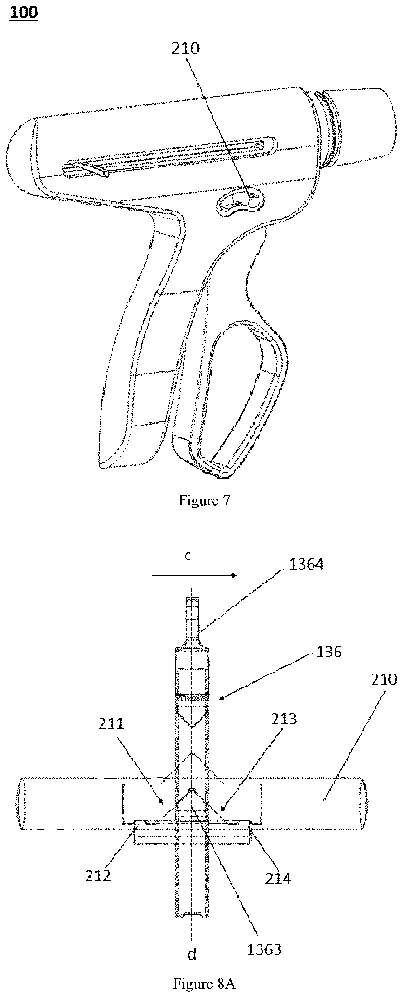

Figure 7 is a schematic diagram of the first operating piece 210 as shown according to some embodiments of the present specification.

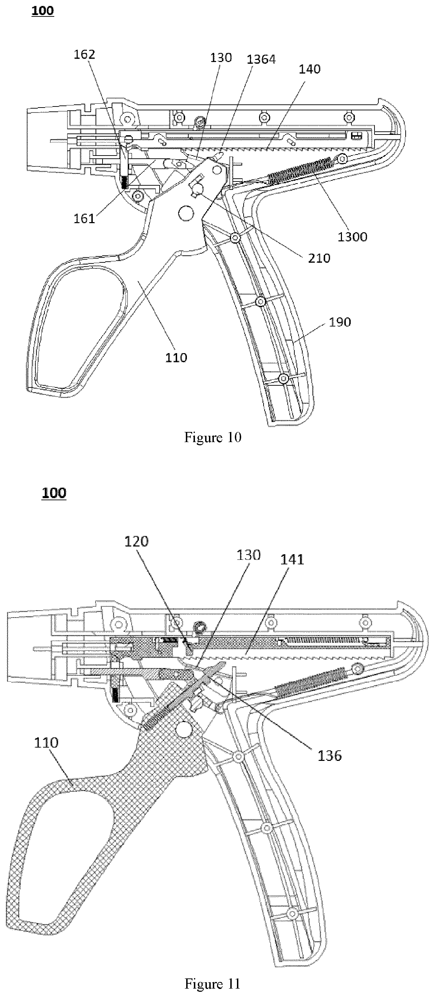

In some embodiments, as shown in Figure 7, the mode switching mechanism may also include a first operating piece 210, which can be pressed and set on the trigger. The first operating piece 210 can be pressed by the operator, thereby enabling functions such as mode switching of surgical suturing instruments. In some embodiments, the first operating piece 210 moves between the initial position and the pressed position. When the first operating piece 210 is pressed and moves from the initial position to the pressed position, the first operating piece 210 drives the switching slider 135 to switch from the first position to the second position, thus enabling the drive claw 130 to switch from the first drive mode to the second drive mode. In some embodiments, the first drive mode can correspond to the end actuator of the surgical suturing instrument performing the first mode, which can be the squeezing mode, where the tool component of the end actuator, such as the jaws, performs a closing operation to squeeze the tissue; The second drive mode can correspond to the end actuator of the surgical suturing instrument performing the second mode, which can be the firing mode, that is, the tool component of the end actuator, such as the cutter and the pinning device, etc., performing the forward direction movement and performing the cutting and suturing operation on the tissue. For more information on the mode switching of surgical suturing instruments achieved by operating the 210 movement of the first operating piece, see Figures 10-19 and their related descriptions, which will not be repeated here.

In some embodiments, the switch between the first drive mode and the second drive mode of the drive claw 130 can be achieved through the operating piece. In the example, the operating piece can be connected to the switching slider 136 and can be operated by the operator to move, thereby driving the switching slider 136 to move between the first and second positions, thus enabling the driving claw 130 to switch between the first and second driving modes. In the example, the operating piece can be connected to the switching slider 136 through a transmission device (such as a rack and pinion, etc.), and by the operator applying a force to the operating piece (if the operating piece is a knob, the operator applies a rotational force to the operating piece), the transmission device converts this force into a force that drives the switching slider 136 to move. This drives the switching slider 136 to move between the first position and the second position, thereby enabling the drive claw 130 to switch between the first drive mode and the second drive mode.

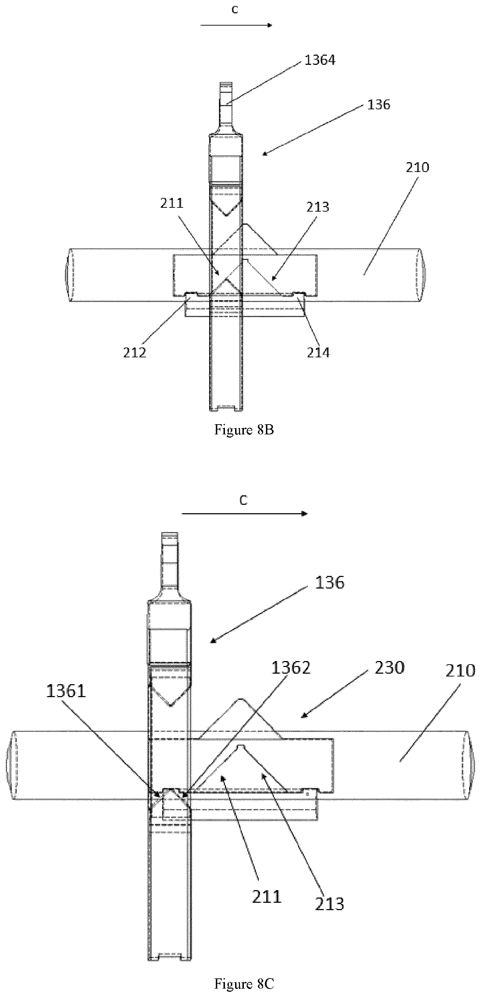

Figure 8A is a schematic diagram of the operation mode of the first operating piece 210 as shown in some embodiments of this specification. Figure 8B is a schematic diagram of the operation mode of the first operating device 210 as shown in some embodiments of the present Specification. Figure 8C is a schematic diagram of the operation mode of the first operating device 210 as shown in some embodiments of this specification.

In some embodiments, as shown in Figure 6, the trigger 110 is provided with a first accommodation slot, and the switching slider 136 can be located within the first accommodation slot. The first accommodation slot can guide the movement of the switching slider 136. In some embodiments, a first elastic element 134 is provided between the switching slider 136 and the bottom of the first accommodation slot. The first elastic element 134 May include, but is not limited to, an elastic element such as a spring or an elastic rubber element. Under the elastic effect of the first elastic element 134, the switching slider 136 can move relative to the trigger 110. In the example, the switching slider 136 can move upwards towards the rack 140 or downwards away from the rack 140. By setting the first elastic member 134, the switching slider 136 can be closely attached to the first operating member 210 to enhance the transmission effect, and under the elastic effect of the first elastic member 134, the first operating member 210 can easily return to its original position.

In some embodiments, the switching slider 136 can be driven and connected to the first operating piece 210. Through the first operating piece 210, the driving claw 130 can be driven to move by the switching slider 136, changing the relative position state between the limit part 1364 and the latch 120, thereby changing the operating mode of the driving structure 100, that is, the working mode corresponding to the switching sutured instrument. In some embodiments, when the first operating piece 210 is pressed and moves from the initial position to the pressed position, the first operating piece 210 drives the switching slider 136 to move from a position close to the rack 140 to a position away from the rack 140, which can drive the driving claw 130 to move downward. This causes the limit part 1364 located within the first limit slot 121 to disengage from the first limit slot 121, even if the drive claw 130 switches from the first drive mode to the second drive mode, thus switching the surgical suture instrument from the first mode to the second mode. It should be noted that the movement of the switching slider 136 from the position close to the rack 140 to the position away from the rack 140 can be from top to bottom. By setting the switching slider 136, the movement direction can be switched, and the force applied by the operator towards the interior of the drive structure 100 can be converted into a force from top to bottom, thereby achieving mode switching. For more on mode switching, see the relevant description below.

There can also be other drive structures between the switching slider 136 and the first operating piece 210. In some other embodiments, the drive structure may include a drive assembly that drives between the switching slider 136 and the first operating piece 210. For example only, the drive assembly may include a first rack, a second rack and a gear, both of which can mesh with the gear. The first rack can extend along the first direction, the second rack can extend along the movement direction of the switching slider 136 (for example, both perpendicular to the rack and perpendicular to the first direction), when the first operating piece 210 is pressed in the first direction, the first rack can drive the gear to rotate, and the gear can drive the second rack to move along the extension direction of the second rack. The second rack can move from a position close to rack 140 to a position away from rack 140, thereby achieving mode switching. The gear can also be replaced with a gear set (that is, two or more gears) to achieve a change in the transmission ratio.

In some embodiments, as shown in Figures 8A, 8B and 8C, the first operating piece 210 May include the first inclined plane 211, with the inclined plane of the first inclined plane 211 facing the first direction (as indicated by arrow c in Figures 8A, 8B and 8C), and the switching slider 136 includes the second inclined plane 1361 that fits with the first inclined plane 211. The second inclined plane 1361 can move along the first inclined plane 211. In some embodiments, components such as the drive claw 130 and rack 140 can be on one side of the first inclined plane 211 (as shown in Figures 7 and 8A, the lower side), the second inclined plane 1361 can be on the other side of the first inclined plane 211 (as shown in Figures 8A and 8B), and in some embodiments, the first inclined plane 211 can be of other shapes, for example, it can be of a curved surface, The second inclined plane 1361 can be an arc that matches it, with no restrictions on the specific shapes of the first inclined plane 211 and the second inclined plane 1361, provided that the second inclined plane 1361 can move along the first inclined plane 211 when the first operating piece 210 is pressed, And be able to drive the switching slider 136 to move near the position of rack 140 and away from the position of rack 140.

In some embodiments, when the first operator 210 is pressed in the first direction and moves from the initial position to the pressed position, the second inclined plane 1361 can move along the first inclined plane 211 (as shown in Figures 8A and 8B, the second inclined plane 1361 moves downward), To drive the switching slider 136 to move from a position close to the rack 140 to a position away from the rack 140, thereby driving the limit part 1364 out of the first limit slot 121, thus achieving mode switching.

In some embodiments, the stroke of the lateral and longitudinal movements can be adjusted by adjusting the Angle between the first inclined plane 211 and the first direction, thereby optimizing the internal space of the device. For example, when the Angle is 45° and the stroke of the lateral and longitudinal movements is 1:1, in order to make the internal structure of the device compact, the smaller stroke of the first operating piece 210 can be achieved by adjusting the Angle, corresponding to the longer longitudinal movement distance of the switching slider 136. In some embodiments, by adjusting the Angle between the first inclined plane 211 and the first direction of the first operating piece 210, the smoothness of pressing the first operating piece 210 can be adjusted to enhance the user experience. In some embodiments, the Angle between the first inclined plane 211 and the first direction of the first operating piece 210 can be set as needed to make the operator's operation more comfortable and improve the operating experience.

In some embodiments, as shown in Figures 8A, 8B and 8C, the first operating piece 210 May include the third inclined plane 213; The switching slider 136 May include a fourth inclined plane 1362 that fits with the third inclined plane 213. In some embodiments, the first inclined plane is associated with the fourth inclined plane 1362 which can move along the third inclined plane 213 when the first operating piece 210 is pressed in the second direction and moves from the initial position to the pressed position; To drive the switching slider 136 to move from a position close to the rack 140 to a position away from the rack 140. Where, the first direction is opposite to the second direction.

In some embodiments, the Angle between the first inclined plane 211 and the first direction can be equal to the Angle between the third inclined plane 213 and the second direction. In this case, the first operating piece 210 can be symmetrical based on the plane where the connection between the first inclined plane 211 and the third inclined plane lies (plane d as shown in Figure 8A). Because of the symmetrical structure of the first operator 210, the operator can press the first operator 210 in the first direction as well as in the second direction to produce the same operation result, which makes it convenient for operators with different operation habits to perform the operation. In some embodiments, the Angle between the first inclined plane 211 and the first direction may not be equal to the Angle between the third inclined plane 213 and the second direction. To achieve the same operational effect, pressing the first operating piece 210 in the first direction and pressing it in the second direction may require different travel distances.

In some embodiments, the first operating piece 210 May include a locking slot, and the switching slider 136 May include a locking structure that cooperates with the locking slot, for example, the locking structure may include a convex column, and when the convex column is inserted into the locking slot, the switching slider 136 and the first operating piece 210 no longer move relative to each other, thus achieving locking. In other embodiments, the locking structure may also include a hook, and when the hook is hooked into the locking slot, the locking is achieved when the switching slider 136 and the first operating piece 210 no longer move relative to each other. In some embodiments, since the first operating piece 210 can be a symmetrical structure, the first operating piece 210 May include a symmetrically set first locking slot 212 and a second locking slot 214, and the locking structure may be coordinated with either the first locking slot 212 or the second locking slot 214 to achieve locking based on the opposite pressing direction of the operator. The first locking slot 212 May be on the side of the first inclined plane 211 away from the third inclined plane 213, and the second locking slot 212 May be on the side of the third inclined plane 213 away from the first inclined plane 211. In some embodiments, the relative motion of the first operating piece 210 and the switching slider 136 is restricted when the first operating piece 210 is pressed and moves to the locking structure fit with the locking slot.

In some embodiments, as shown in Figure 7, the first operator 210 May have a rod-shaped structure to facilitate the operator's pressing. In some embodiments, the first operating piece 210 May include the mating part 230, the first inclined plane 211 and the third inclined plane 213 (see Figure 8C) may be set on the mating part 230, and a cavity may be formed between the first inclined plane 211 and the third inclined plane 213. The second inclined plane 1361 and the fourth inclined plane 1362 on the switching slider 136 can be set in the cavity, and the second inclined plane 1361 and the fourth inclined plane 1362 of the switching slider 136 can come into contact with the first inclined plane 211 and the third inclined plane 213 respectively. In some embodiments, the first locking slot 212 and the second locking slot 214 can both be set on the mating part 230 (on the lower surface of the mating part 230 as shown in Figure 8C). In some embodiments, a drive boss 1363 can be set on the switching slider 136, and both the second slope 1361 and the fourth slope 1362 can be set on the drive boss 1363.

The relative motion of the first operating piece 210 and the switching slider 136 is further described in conjunction with Figures 8A, 8B and 8C. It should be noted that the following description is used as an example rather than a limitation of the embodiments of this specification.

As shown in Figure 8A, the first operator 210 is in the initial position, at which point the second inclined plane 1361 of the switching slider 136 is in full contact with the first inclined plane 211, and the second inclined plane 1361 is at the top of the first inclined plane 211. As shown in Figure 8B, the operator presses the first operator 210 in the first direction (direction c in Figure 8B), and the second inclined plane 1361 moves along the first inclined plane 211, which drives the switching slider 136 to move downward, causing the switching slider 136 to move from a position close to rack 140 to a position away from rack 140. As shown in Figure 8C, with the operator's press, the second inclined plane 1361 moves to the bottom of the first inclined plane 211 and can no longer move along the inclined plane, then as the first operating piece 210 continues to move in the first direction, the locking structure of the switching slider 136 moves into the first locking slot 212 of the first operating piece 210. At this point, the relative motion between the first operating piece 210 and the switching slider 136 is locked, and the first operating piece 210 is in the pressed position, allowing the drive claw 130 to maintain the second switching mode.

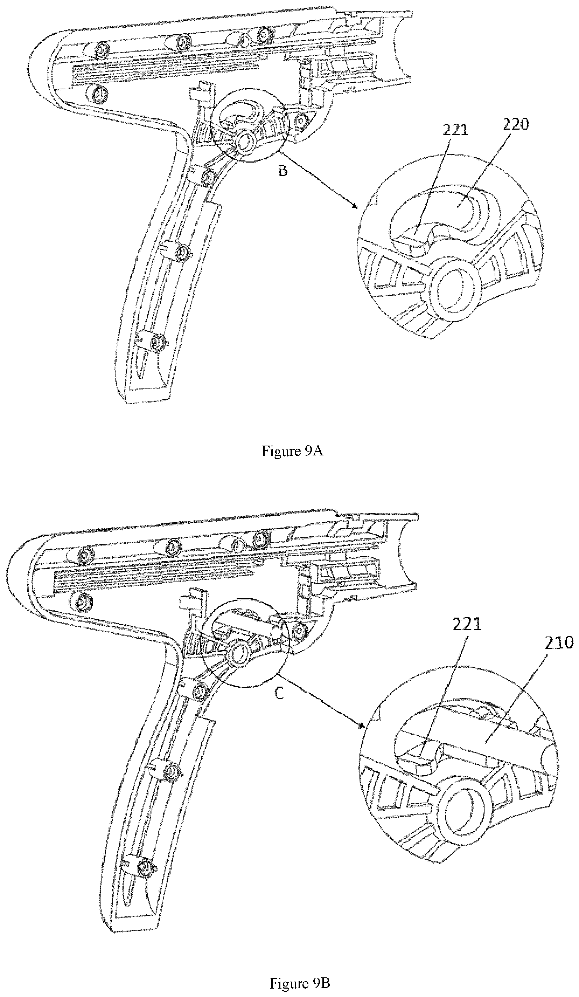

Figure 9A is a structural diagram of the button hole 220 as shown according to some embodiments of this specification. Figure 9B is a schematic diagram of the structure of the button hole 220 as shown in some embodiments of this specification.

In some embodiments, as shown in Figures 9A and 9B, the drive structure 100 further includes a button hole 220, with the first operating piece 210 slidably arranged within the button hole 220, and the button hole 220 includes a button reset section 221 that cooperates with the first operating piece 210. The button reset section 221 is used to apply a force to the first operating piece 210 in a direction opposite to the pressing direction; The trigger 110 resets under the action of the pull spring 1300. When the trigger 110 resets, the first operating piece 210 slides within the button hole 220 while the first operating piece 210 moves in the opposite direction to the pressing under the action of the button reset part 221. In some embodiments, the button reset section 221 can be set at the lower part of the button hole 220. In some embodiments, corresponding to the press position of the first operating piece 210, the button reset part 221 May not completely cover the lower part of the button hole 220; Corresponding to the initial position of the first operating piece 210, the button reset section 221 May fully cover the lower part of the button hole 220; There can be a smooth connection between the incompletely covered button reset section 221 and the fully covered button reset section 221. When pulling the spring 1300, the first operating piece 210 can be gradually restored from the pressed position to the initial position under the action of the button reset part 221.

In some embodiments, as shown in Figure 2, the drive structure 100 May also include a pull-back structure for pulling the rack 140 and the tool assembly of the end actuator connected to the rack 140 back to the initial position, which may correspond to the positions of the components of the surgical suturing instrument when the end actuator performs the initial mode. In some embodiments, the pull-back structure may include a pull-back button 180 and a stop plate 170. The pull back knob 180 is used to pull the rack 140 in the opposite direction of its forward movement. The stop plate 170 can be used to cover the teeth of the rack 140 from the side of the rack 140, so that under the action of the stop plate 170, the drive claw 130 can be pressed down, causing the rack 140 to disconnect from the drive claw 130, at which point the rack 140 is equivalent to a smooth long strip that can be easily pulled back. In some embodiments, the pull back knob 180 can be exposed outside the handle housing 190 to allow the operator to pull it back.

In some embodiments, the side of the rack 140 (the side facing the viewer as shown in Figure 2) is provided with a protrusion 171, and the stop plate 170 May be provided with a chute that fits with the protrusion 171, and the protrusion 171 can be clamped into the chute, thereby setting the stop plate 170 on the side of the rack 140, and the pull back button 180 can be connected to the stop plate 170. In some embodiments, when no external force is applied to the stop plate 170, the stop plate 170 does not cover the teeth of the rack 140, and the rack 140 can still perform its transmission function. In some embodiments, when the stopper 170 is moved backward of the rack 140 by pulling back the knob 180, based on the guiding effect of the chute, the stopper 170 can move downward, thereby pressing down the drive claw 130 and the stopper slider 162, making the rack 140 equivalent to a smooth long strip. It is impossible to fit with the drive claw 130 and the anti-retreat slider 162 by clamping, so that the rack 140 can be pulled back to the original position of the rack 140.

In some embodiments, based on the rack 140 being pulled back to the initial position of the rack 140, the tool assembly of the end actuator connected to the rack 140, for example, the cutter and the pinning device, can be pulled back to the initial position together. In some embodiments, the pullback button 180 can be functionally connected to the end actuator, and when pulled back, the tool components of the end actuator, e.g., the cutter and the nailing device, do not perform cutting and sewing operations; Further, after being pulled back to the initial position, the tool assembly of the end actuator, e.g., the jaws, etc., can be opened to release the tissue. At the same time, trigger 110 resets under the action of pull spring 1300, with trigger 110 at the farthest end. When used again, by pulling the trigger 110, the drive claw 130 moves forward, allowing the working components of the end actuator to perform corresponding operations, such as closing the jaws, cutting the cutter and the pinning device for cutting and sewing. For more information on the operation of the end actuator through the drive structure 100, see the relevant description below.

Some embodiments of this specification provide a surgical suture instrument, which may include a handle part, an end actuator and a drive structure of any of the above embodiments 100. In some embodiments, the drive structure 100 is arranged within the handle housing 190 of the handle unit, and the drive structure 100 includes an operable trigger 110 and an operable first operating piece 210, etc., for the operator to operate by hand, and the trigger can be rotatably arranged on the handle housing 190. The handle housing 190 May be provided with a through hole for threading the first operating piece 210, and the button hole 220 May be opened on the handle housing 190. The end actuator may include a tool assembly with clamping, cutting and sewing functions, for example, a jaws, a cutter and a nailing device. The drive structure 100 can be set inside the handle unit, and the drive structure 100 includes movable rack 140, etc. The end actuator can be connected to the drive structure 100, for example, the cutter and nailing device of the end actuator can be connected to the rack 140. In some embodiments, the end actuator has multiple working modes, and different working modes can be controlled and executed through the drive structure 100 based on the operation of the handle unit, and the operation of the handle unit can also be adjusted through the drive structure 100 to adjust the working mode of the end actuator. More information on adjusting the working mode of the end actuator through the drive structure can be found in the relevant description below.

In some embodiments, the end actuator has multiple executable working modes, and the operator can select the appropriate mode based on clinical needs. In some embodiments, surgical suturing devices may have initial mode, first mode, and second mode. The initial mode may correspond to the initial mode of the surgical suturing instrument, which corresponds to the original state of the surgical suturing instrument in which no work has been performed. The first mode corresponds to the compression mode of the surgical suture instrument. Pressing the trigger 110 corresponds to the gradual closing of the jaws of the end actuator, clamping and further squeezing the tissue between the jaws. In this mode, pressing the trigger 110 can be used to achieve tissue compression, and the drive structure 100 does not need to drive other tool components (such as cutters, etc.) forward to perform operations such as cutting. The second mode corresponds to the firing mode of the surgical suturing instrument, which may include pushing nail shaping and pushing knife cutting, etc. The driving structure 100 needs to drive the related components such as rack 140 forward, so that the tool components of the end actuator connected to rack 140, such as the cutter and the pinning device, can perform operations such as cutting and suturing simultaneously. The following illustrates the working mode of the surgical suturing instrument through some embodiments. It should be noted that the following is only an example and not a limitation to this specification. The surgical suturing instrument and its driving structure 100 in the embodiments of this specification can be used in any other feasible way of use.

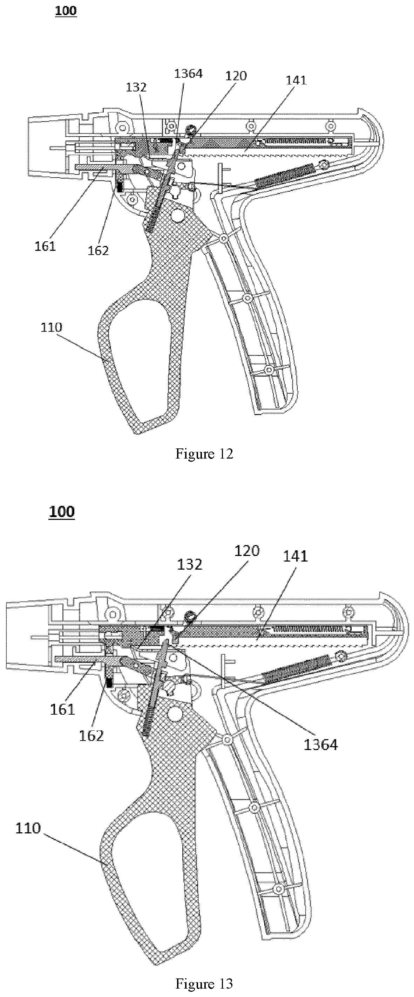

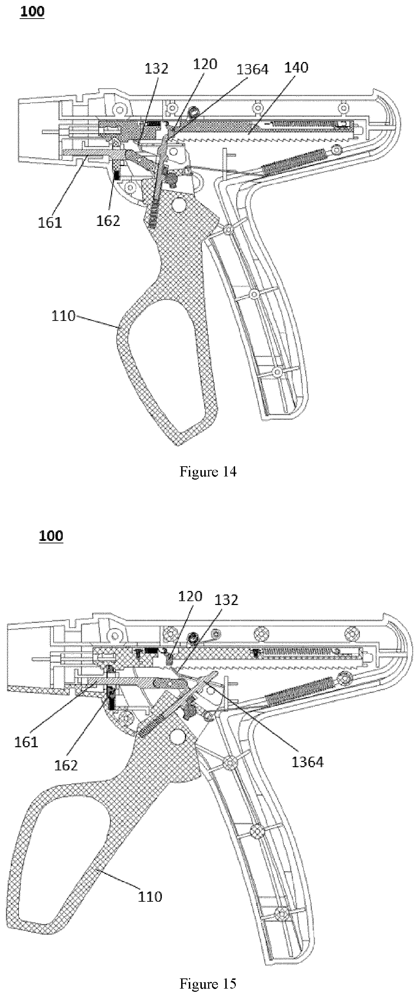

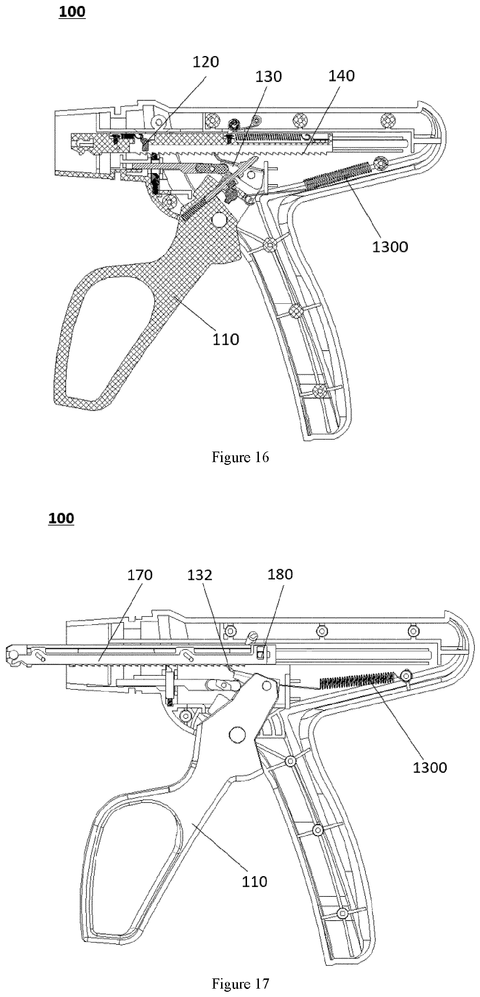

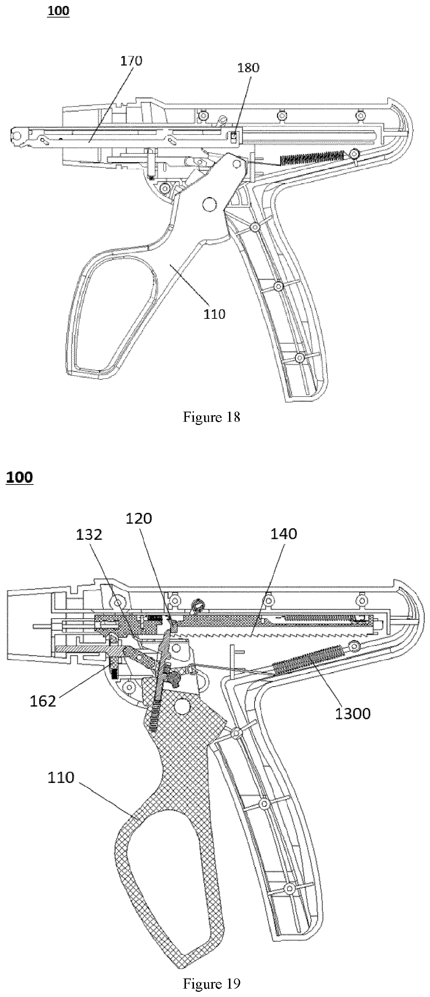

Figure 10 is a schematic diagram of the initial mode shown according to some embodiments of this specification. Figure 11 is a schematic diagram of the first pattern as shown according to some embodiments of this specification. Figure 12 is a schematic diagram of the first pattern as shown according to some embodiments of this specification. Figure 13 is a schematic diagram of the first pattern as shown according to some embodiments of this specification. Figure 14 is a schematic diagram of the mode switching as shown according to some embodiments of this specification. Figure 15 is a schematic diagram of the second mode as shown according to some embodiments of this specification. Figure 16 is a schematic diagram of the second pattern as shown according to some embodiments of this specification. Figure 17 is a schematic diagram of resetting to the initial mode as shown in some embodiments of this specification. Figure 18 is a schematic diagram of resetting to initial mode as shown in some embodiments of this specification. Figure 19 is a schematic diagram of resetting to initial mode as shown in some embodiments of this specification.

As shown in Figure 10, in some embodiments, the position of the rack 140 that drives structure 100 is locked when the end actuator performs the initial mode. In the example, the anti-retreat slider 162 is lifted by the elasticity of the spring and holds the rack 140 against it on the forward path of the rack 140, restricting the forward movement of the rack 140; The drive claw 130 is located at the rear side of the latch 120, and the drive claw 130 is against the rack 140; The switching slider 136 does not touch the rack 140; The trigger 110 can be applied to the rearward direction of the rack 140 under the action of the spring 1300, and the external handle housing 190 can be correspondingly set with the convex bar, and the trigger 110 can be pulled by the spring 1300 against the convex bar, at which point the trigger 110 is in the maximum Angle position.

In some embodiments, the movement of the rack 140 that drives the structure 100 is restricted when the end actuator performs the first mode. In the example, at the starting position, the stop slider 162 is pushed up to limit the advance of the rack 140, at which point the trigger 110 is at the farthest end and the drive claw 130 is behind the latch 120, as shown in Figure 11. Pull the trigger 110, and the trigger 110 drives the drive claw 130 forward, switching the limit part 1364 of the slider 136 to push the latch 120 to rotate; When the trigger 110 is engaged to the set position, the limit part 1364 drives the latch 120 to rotate beyond the limit position, and the latch 120 returns to the initial position, then the latch 120 bounches to the rear side of the drive claw 130, at which point the limit part 1364 of the switching slider 136 is in the first limit slot 121; Continuing to pull the trigger 110, the stop slider 162 is pushed down by the protrusion of the forward slider 161 and begins to release the limit on the rack 140; At this point, the drive claw 130 has not yet come into contact with the rack 140 and has not yet started pushing the rack 140, as shown in Figure 12. The drive claw 130 is pushed forward by the trigger 110, and the end of the drive claw 130's claw 132 touches the rack step of the rack 140. At this point, the stop slider 162 has been completely pushed down, completely removing the restriction on the rack 140; Pull the trigger 110 further to the nearest end, and the rack 140 moves forward under the push of the end of the drive claw 130's claw 132, thereby enabling the end actuator of the surgical suture instrument to perform the jaws closure operation to complete the squeezing action on the tissue, as shown in Figure 13. In some embodiments, the trigger 110 is turned to the distal end, and the limit part 1364 can be pressed against the latch 120, thereby pushing the rack 140 to move backward, which in turn enables the end actuator of the surgical suture instrument to perform the opening of the jaws. Since the limit part 1364 of the switching slider 136 remains within the first limit slot 121 throughout the process and does not disengage, the movement of the rack 140 is controlled by the movement of the trigger 110. Based on the movement of the trigger 110 to drive the claw 130 and the limit part 1364, the rack 140 can move forward and backward within a certain range. This enables the closing and opening of the actuator.

In some embodiments, the operator can switch the mode of the surgical suture instrument through the first operating piece 210, that is, switch from the first mode to the second mode. In the example, as shown in Figure 14, the operator can release the trigger 110, which automatically returns to the initial position under the action of the pull spring 1300, and eventually the trigger 110 and the drive claw 130 are limited by the latch 120; The operator can press the first operating piece 210, under the action of the inclined plane, the switching slider 136 will move the limit part 1364 by driving it out of the first limit slot 121. When the limit part 1364 is completely out of the first limit slot 121, at this time the drive claw 130 will be together with the trigger 110 under the action of the tension spring 1300, Move the rack 140 backward to the rear side of the latch 120; Under the action of the tension spring 1300 and the button reset part 221 of the button hole 220, the first operating piece 210 returns to its original position.

In some embodiments, when the end actuator performs the second mode, the rack 140 of the drive structure 100 is able to move forward. In the example, the end actuator performs the second mode when the claw end 132 of the drive claw 130 holds against the teeth of the rack 140; The operator pulls the trigger 110 and pushes the rack 140 through the drive claw 130 to advance the rack 140, as shown in Figure 15. In this mode (and in other cases except the squeeze mode), the limit part 1364 can be located within the avoidance slot 141 of the rack 140, thus not affecting the movement of the rack 140. In some embodiments, the tool assembly of the surgical suturing instrument attached to the rack 140, for example, the cutter and the stitching device, can achieve the cutting and suturing of the tissue based on the advance of the rack 140; The operator releases the trigger 110, and the drive claw 130, along with the trigger 110, can retreat under the action of the pull spring 1300, and the anti-retreat slider 162 is bounced up under the action of the spring element to hold against the teeth of the rack 140, thereby limiting the retreat of the rack 140, as shown in Figure 16. When the rack 140 has completed the full stroke (corresponding to after the user has finished sewing), the drive claw 130 and the anti-retreat slider 162 can hold the teeth of the rack 140 to limit the backswing of the rack 140, as shown in Figure 17; The operator manually moves the pull back knob 180 in the backward direction of the rack 140, and the stop plate 170 responds to the pull back of the pull back knob and moves downward based on the guiding effect of the groove to hold against the drive claw 130 and the stop slider 162, thereby releasing the engagement of the drive claw 130 and the stop slider 162 with the rack 140, and thus the rack 140 can be pulled back, as shown in Figure 18. This corresponds to the opening of the end actuator jaws.

In some embodiments, while the rack 140 is pulled back, the trigger 110 resets under the action of the pull spring 1300. When the trigger 110 resets, the first operating piece 210 slides within the button hole 220 while the first operating piece 210 moves in the opposite direction to the pressing direction under the action of the button reset part 221. This achieves the resetting of the first operating piece 210; At this point, the end actuator performs the initial mode, the position of the rack 140 is locked by the stop slider 162, the drive claw 130 is on the rear side of the latch 120, the drive claw 130 is against the rack 140, the switching slider 136 is not in contact with the rack 140, and the trigger 110 is pulled by the pull spring 1300 against the convex strip of the handle housing 190 under the action of the pull spring 1300. Pull the trigger 110 and the end actuator begins to execute the first mode. For more details on executing the first mode, see the relevant description above.

In some cases, if you are not satisfied with the area of tissue squeezing and the operator does not press the mode switch button, that is, the first operating piece 210, trigger 110 can be operated in the opposite direction of the clamping, causing the drive claw 130 to move backward towards the rack 140, thereby causing the latch 120 to respond to the movement of the drive claw 130 and drive the rack 140 to move backward. Figure 19 shows that. In some embodiments, the backward movement of rack 140 can cause the end actuator to open, achieving the release of the tissue.

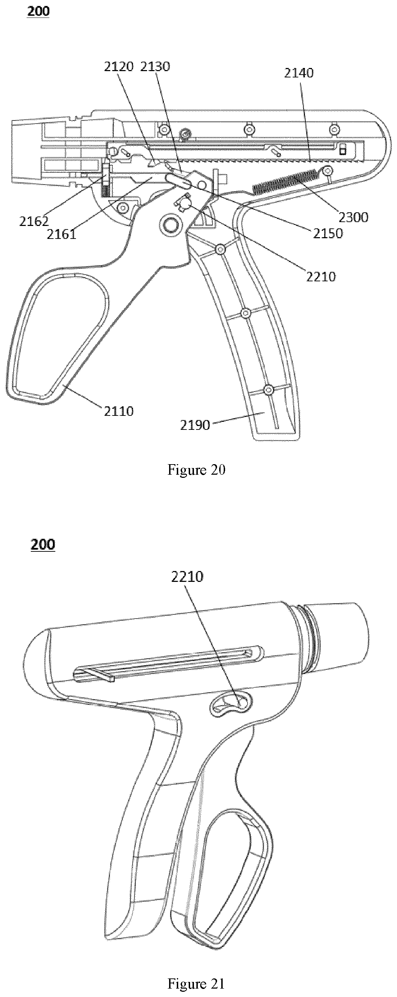

Figure 20 is a structural diagram of the drive structure 200 as shown in some other embodiments of the present Specification.

In some embodiments, as shown in Figure 20, the drive structure 200 May be set in the handle housing 2190 of the surgical suture instrument, and the drive structure 200 May include a trigger 2110, a rack 2140, a drive claw 2130 and a mode switching mechanism. In some embodiments, the mode switching mechanism may include the latch 2120, the drive assembly and the operation assembly. The operating component includes a second operating piece 2210, which is pressable and set on the trigger 2110, and moves between the initial position and the pressed position when the second operating piece 2210 is pressed and moves from the initial position to the pressed position, The second operator 2210 drives the drive claw 2130 to switch from the first drive model to the second drive mode. In some embodiments, in the first drive mode, the drive claw 2130 cooperates with the latch 2120, and the latch 2120 is confined within the preset travel. In some embodiments, the stroke range of the preset stroke is related to the extent to which the latch 2120 is restricted by the drive claw 2130, for example, the latch 2120 is restricted in the third limit slot of the drive claw 2130, and the distance that the latch 2120 can move within the third limit slot corresponds to the preset stroke. In some embodiments, the latch 2120 is confined in the third limit slot of the drive claw 2130, and the trigger 2110 drives the drive claw 2130 to move, thereby enabling the latch 2120 located in the third limit slot of the drive claw 2130 to move, allowing the rack 2140 to move forward or backward within a certain stroke. In some embodiments, in the first drive mode, the surgical suture instrument corresponds to the execution of the first mode (e.g., the squeeze mode). In some embodiments, in the second drive mode, the latch 2120 disengages from the drive claw 2130, and the surgical suturing instrument can perform the second mode (such as the firing mode). The following will provide a detailed description of the components of the drive structure 200, and it should be noted that the following embodiments are for illustrative illustration only of the implementation of the drive structure 200 and its components.

Trigger 2110 has the same structure as trigger 110 in drive structure 100 and will not be repeated.

The structure of the latch 2120 is the same as that of the latch 3120 in the drive structure 300 below, the structure of the drive claw 2130 is the same as that of the drive claw 3130 below, and the fit of the latch 2120 with the drive claw 2130 is the same as that of the latch 3120 with the drive claw 3130, see below and will not be elaborated further.

The rack 2140, spring 2300, forward slider 2161, anti-slip slider 2162, link 2150 and pull back structure are the same as the rack 140, spring 1300, forward slider 161, anti-slip slider 162, link 150 and pull back structure in drive structure 100, and will not be elaborated here.

Figure 21 is a schematic diagram of the second operating piece 2210 as shown according to some other embodiments of the present specification.

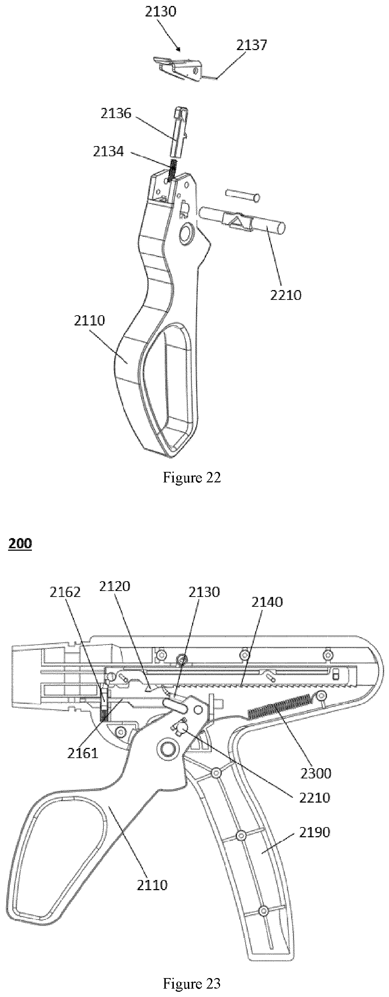

The second operating piece 2210 can be pressed by the operator, thereby achieving functions such as mode switching of surgical suture instruments. In some embodiments, as shown in Figure 21, the second operating piece 2210 moves between the initial position and the pressed position. When the second operating piece 2210 is pressed and moves from the initial position to the pressed position, the second operating piece 2210 drives the drive claw 2130 to switch from the first drive mode to the second drive mode. In some embodiments, in the first drive mode, the drive claw 2130 cooperates with the latch 2120, and the latch 2120 is confined within the preset travel; In the second drive mode, the latch 2120 disengages from the drive claw 2130. In some embodiments, the first drive mode can correspond to the end actuator of the surgical suturing instrument performing the first mode, which can be the squeezing mode, where the tool component of the end actuator, such as the jaws, performs a closing operation to squeeze the tissue; The second drive mode can correspond to the end actuator of the surgical suturing instrument performing the first mode, and the second mode can be the firing mode, that is, the tool component of the end actuator, such as the cutter and the pinning device, etc., performing the forward direction movement, performing the cutting and suturing operation on the tissue. More information on the mode switching of surgical suturing instruments by operating the 2210 movement of the second operating piece can be found in Figures 23-32 and their related descriptions, which will not be repeated here.

Figure 22 is a schematic diagram of the installation of the second operator 2210 as shown in some other embodiments of this specification.

In some embodiments, as shown in Figure 22, the drive assembly includes a drive slider 2136 that is transmitted and connected between the drive claw 2130 and the second operating piece 2210. In some embodiments, the drive slider 2136 can be set on the trigger 2110, and the drive slider 2136 can drive the drive claw 2130 to move, changing the relative position state of the drive claw 2130 to the latch 2120, thereby changing the operating mode of the drive structure 200, that is, the working mode corresponding to the switching of the sewing instrument. In some embodiments, when the second operating piece 2210 is pressed and moves from the initial position to the pressed position, the second operating piece 2210 drives the driving slider 2136 to move from the position close to the rack 2140 to the position away from the rack 2140, which can drive the driving claw 2130 to move downward. This causes the latch 2120 located in the third limit slot of the drive claw 2130 to disengage from the third limit slot, even if the drive claw 2130 switches from the first drive mode to the second drive mode, causing the rack 2140 to switch from the first mode to the second mode. It should be noted that the movement of the drive slider 2136 from a position close to rack 2140 to a position away from rack 2140 can be from top to bottom as shown in Figure 21. By setting the drive slider 2136, it is possible to switch the direction of motion, converting the force applied by the operator towards the interior of the drive structure 200 into a force from top to bottom, thereby driving the drive claw 2130 to move downward and achieve the drive mode switch. For more information on mode switching, see the relevant description below.

In some embodiments, the second operating piece 2210 includes the first inclined plane and the third inclined plane, and the drive slider 2136 includes the second inclined plane and the fourth inclined plane. The structures of the first inclined plane, the second inclined plane, the third inclined plane and the fourth inclined plane are the same as those of the first inclined plane 211, the second inclined plane 1361, the third inclined plane 213 and the fourth inclined plane 1362 in the drive structure 100. No further elaboration is needed.

In some embodiments, a second accommodation groove is provided on the trigger 2110, the drive slider 2136 is provided within the second accommodation groove, and a second elastic member 2134 is provided between the drive slider 2136 and the bottom of the second accommodation groove. The second accommodation slot can act as a guide for the movement of the drive slider 2136. In some embodiments, a second elastic element 2134 is provided between the drive slider 2136 and the bottom of the second accommodation groove. The second elastic element 2134 May include, but is not limited to, a spring, an elastic rubber element, etc. Under the elastic effect of the second elastic element 2134, the drive slider 2136 can move relative to the trigger 2110. For example, The drive slider 2136 can either move upward towards the rack 2140 or downward away from the rack 2140. By setting the second elastic member 2134, the drive slider 2136 can be closely attached to the second operating member 2210 to enhance the drive effect, and under the elastic effect of the second elastic member 2134, the second operating member 2210 can easily return to its original position.

In some embodiments, the second operating piece 2210 includes a locking slot, and the drive slider 2136 includes a locking structure that cooperates with the locking slot; When the second operating piece 2210 is pressed and moves to the point where the locking structure fits with the locking slot, the relative motion of the second operating piece 2210 and the driving slider 2136 is restricted. The structure and fit of the locking slot and the locking structure are the same as those of the locking slot and the locking structure in the drive structure 100, and will not be elaborated further.

In some embodiments, as shown in Figure 22, the drive claw 2130 is provided with a drive pin 2137, the drive slider 2136 is provided with a hole, and the drive pin 2137 is movably inserted through the hole of the drive slider 2136. In some embodiments, the hole diameter of the drive slider 2136 is larger than the diameter of the drive pin 2137, allowing the drive pin 2137 to have a certain free movement space within the hole, that is, when the drive pin 2137 is not in direct contact with the drive slider 2136, the drive pin 2137 and the drive slider 2136 can move independently. The holes on the drive slider 2136 can be set with different diameters and shapes as needed, such as rectangular, triangular, circular, polygonal, etc., without any restrictions here. In some embodiments, the drive slider 2136 can be movably connected to the drive claw 2130 through the drive pin 2137 to drive the drive claw 2130 to move, and the drive claw 2130 can be rotatably connected to the trigger 2110.

In some embodiments, the drive pin 2137 can be replaced by other structures that can achieve the same or similar effect, for example, a flexible piece can be arranged between the drive slider 2136 and the drive claw 2130 to achieve the movable connection.

The drive structure 200 also includes a button hole and a button reset unit, which have the same structure as the button hole 220 and button reset unit 221 in the drive structure 100, and will not be elaborated further.

Some embodiments of this specification provide a surgical suture instrument which may include a handle unit, an end actuator, and a drive structure 200 of any of the above embodiments. The end actuator may include a tool assembly with the functions of clamping, cutting and suturing, for example, a jaws, a cutter and a nailing device. The end actuator can be connected to the drive structure 200, for example, the cutter and nailing device of the end actuator can be connected to the rack 2140. In some embodiments, the end actuator has multiple working modes, and different working modes can be controlled and executed through the drive structure 200 based on the operation of the handle unit, and the operation of the handle unit can also be adjusted through the drive structure 200 to adjust the working mode of the end actuator. More information on adjusting the working mode of the end actuator through the drive structure 200 can be found in the relevant description below.

In some embodiments, the end actuator has multiple executable working modes, and the operator can select the appropriate mode based on clinical needs. In some embodiments, surgical suturing devices may have initial mode, first mode, and second mode. The initial mode may correspond to the initial mode of the surgical suturing instrument, which corresponds to the original state of the surgical suturing instrument in which no work has been performed. The first mode corresponds to the compression mode of the surgical suturing instrument. Pressing the trigger 2110 corresponds to the gradual closure of the jaws of the end actuator, clamping and further squeezing of the tissue between the jaws. In this mode, pressing the trigger 2110 can be used to achieve tissue compression. Drive structure 200 does not need to drive other tool components (such as cutters, etc.) forward to perform operations such as cutting. The second mode can correspond to the firing mode of surgical suturing instruments, which can include push nail forming and push knife cutting, etc. The drive structure 200 needs to drive related components such as rack 2140 forward, so that the tool components of the end actuator connected to rack 2140, such as the cutter and the pinning device, perform operations such as cutting and suturing at the same time. The following illustrates the working mode of the surgical suture instrument through some embodiments. It should be noted that the following is only an example and not a limitation to this specification. The surgical suture instrument and its drive structure 200 in the embodiments of this specification can be used in any other feasible way of use.

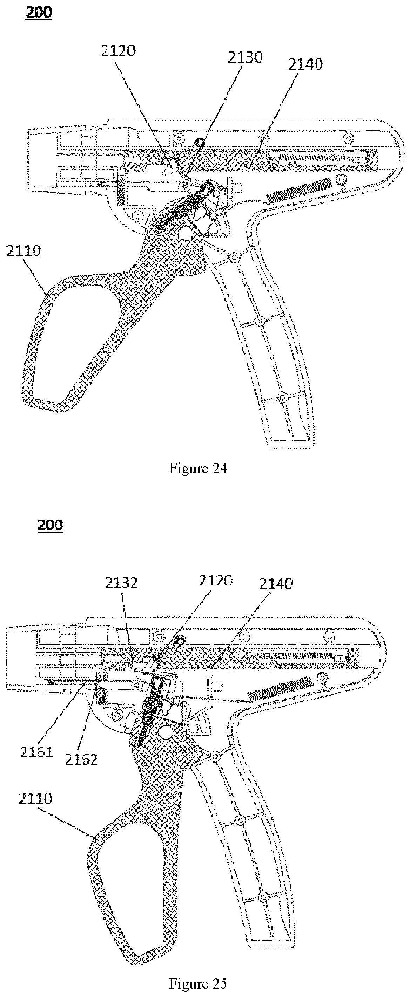

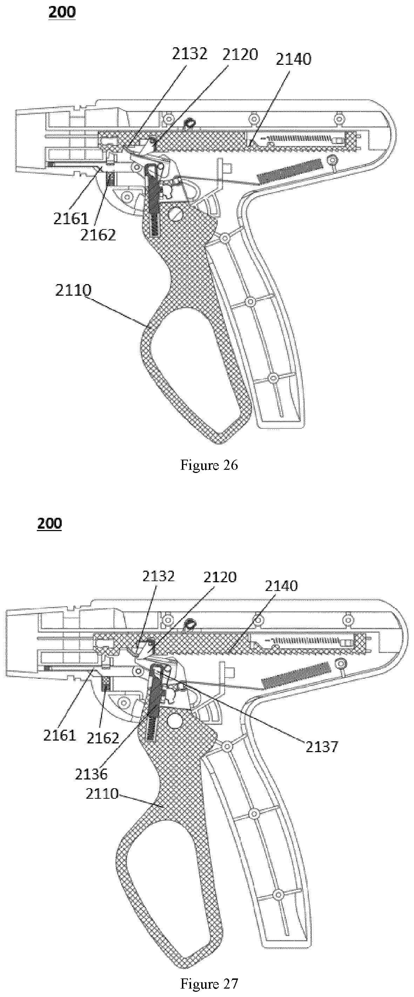

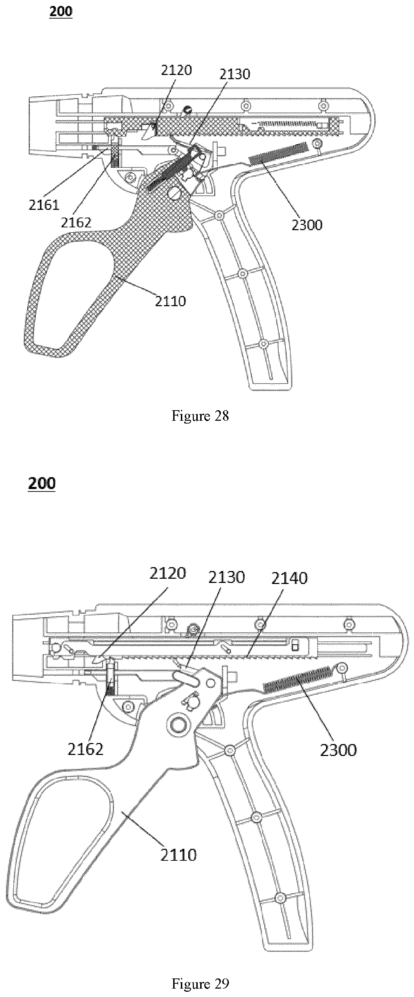

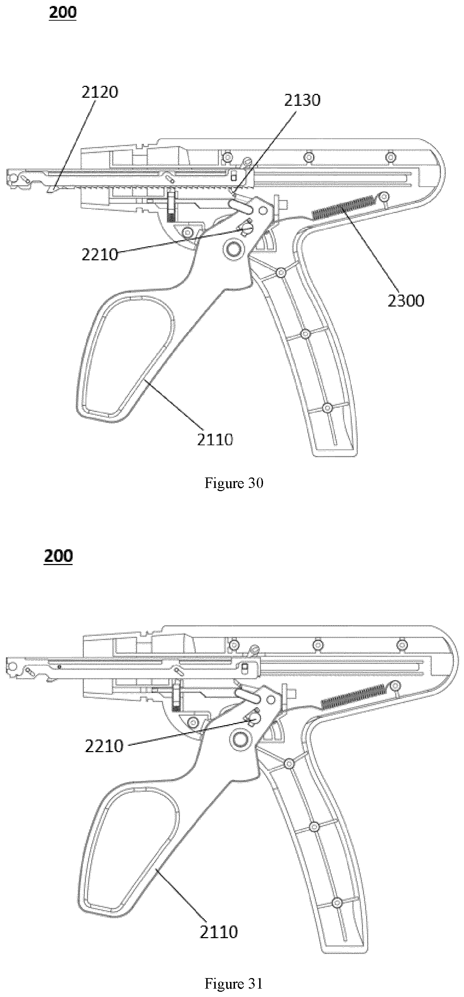

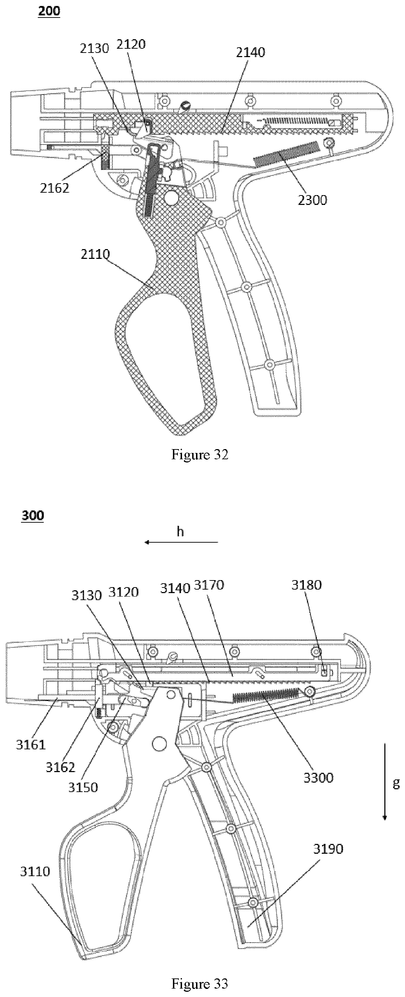

Figure 23 is a schematic diagram of the initial mode as shown according to some other embodiments of this specification. Figure 24 is a schematic diagram of the first pattern as shown according to some other embodiments of this specification. Figure 25 is a schematic diagram of the first pattern as shown according to some other embodiments of this specification. Figure 26 is a schematic diagram of the first pattern as shown according to some other embodiments of this specification. Figure 27 is a schematic diagram of the mode switching as shown according to some other embodiments of this specification. Figure 28 is a schematic diagram of the second mode as shown according to some other embodiments of this specification. Figure 29 is a schematic diagram of the second pattern as shown according to some other embodiments of this specification. Figure 30 is a schematic diagram of resetting to the initial mode as shown in some other embodiments of this specification. Figure 31 is a schematic diagram of resetting to initial mode as shown in some other embodiments of this specification. Figure 32 is a schematic diagram of resetting to initial mode as shown in some other embodiments of this specification.

As shown in Figure 23, in some embodiments, the position of the rack 2140 that drives structure 200 is locked when the end actuator performs the initial mode. In the example, the anti-retreat slider 2162 is lifted by the elasticity of the spring and holds the rack 2140 against it on the forward path of the rack 2140, restricting the forward movement of the rack 2140; The drive claw 2130 is located at the rear side of the latch 2120, and the drive claw 2130 and the latch 2120 respectively hold the rack 2140 under the action of their respective torsion springs; The drive slider 2136 is lifted by the elasticity of the second elastic member 2134, allowing the second operating member 2210 to be in the initial position; The trigger 2110 can be applied to the rearward direction of the rack 2140 under the action of the pull spring 2300, and the handle housing 2190 can be set with the corresponding convex bar, and the trigger 2110 can be pulled by the pull spring 2300 against the convex bar.

In some embodiments, the movement of the rack 2140 that drives the structure 200 is restricted when the end actuator performs the first mode. In the example, at the starting position, the stop slider 2162 is pushed up to limit the advance of the rack 2140, at which point the trigger 2110 is at the farthest end and the drive claw 2130 is behind the latch 2120, as shown in Figure 24; Pull the trigger 2110, the trigger 2110 drives the drive claw 2130 forward, and the end of the claw of the drive claw 2130, 2132, pushes the latch 2120 to rotate; When the trigger 2110 is engaged to the set position, the drive claw 2130 disengages from the latch 2120, and the latch 2120 returns to its initial position, then the latch 2120 bounces into the third limit slot of the drive claw 2130; Continuing to pull the trigger 2110, the stop slider 2162 is pushed down by the bump of the forward slider 2161 and begins to release the limit on the rack 2140, as shown in Figure 25; Continuing to pull the trigger 2110, the stop slider 2162 is gradually pushed down, the drive claw 2130 is driven forward by the trigger 2110, and the end of the claw of the drive claw 2130, 2132, touches the rack step of the rack 2140. At this point, the stop slider 2162 has been fully pushed down, completely lifting the limit on the rack 2140; As shown in Figure 26, further pull the trigger 2110 to the nearest end, and the rack 2140 moves forward under the push of the end of the drive claw 2130's claw 2132, thereby enabling the end actuator of the surgical suture instrument to perform the clamping opening closure operation to complete the squeezing action on the tissue. Since the latch 2120 remains in the third limit slot of the drive claw 2130 throughout the process without disengagement, the movement of the rack 2140 is controlled by the movement of the trigger 2110. Based on the movement of the drive claw 2130 driven by the trigger 2110, the rack 2140 can move forward and backward within a certain range, thereby closing and opening the upper actuator.

In some embodiments, the operator can switch the mode of the surgical suturing instrument through the second operating piece 2210, that is, from the first mode to the second mode. In the example, as shown in Figure 27, the operator can release the trigger 2110, which automatically returns to the initial position under the action of the pull spring 2300, and eventually the trigger 2110 and the drive claw 2130 are locked to the limit by the latch 2120; The operator can release the latch 2120 from the third limit slot of the drive claw 2130 by pressing the second operating piece 2210 in the first direction. Under the action of the inclined plane, the drive slider 2136 drives the drive claw 130 to move through the drive pin 2137, and when the drive claw 2130 is completely released from the latch 2120, At this point, no structure limits the drive claw 2130, and at this point the drive claw 2130 will move along with the trigger 2110, under the action of the pull spring 2300, in the backward direction of the rack 2140 to the rear side of the latch 2120; Drive claw 2130 holds against the teeth of rack 2140; Under the action of the tension spring 2300 and the button hole, the second operating piece 2210 returns to its initial position.

In some embodiments, when the end actuator performs the second mode, the rack 2140 of the driving structure 200 is capable of performing forward motion. In the example, the end actuator performs the second mode when the drive claw 2130 holds against the teeth of the rack 2140; The operator pulls the trigger 2110 and pushes the rack 2140 through the drive claw 130 to advance the rack 2140, as shown in Figure 28. In some embodiments, the tool component of the surgical suturing instrument connected to the rack 2140, for example, the cutter and the pinning device, can achieve cutting and suturing of the tissue based on the advance of the rack 2140; The operator releases the trigger 2110, and the drive claw 2130, along with the trigger 2110, can retreat under the action of the pull spring 2300, and the anti-retreat slider 2162 is bounced up under the action of the spring element to hold against the teeth of the rack 2140, thereby limiting the retreat of the rack 2140, as shown in Figure 29. When the rack 2140 has completed the full stroke (corresponding to the operator's completion of stitching), the drive claw 2130 and the anti-retreat slider 2162 can press against the teeth of the rack 2140 to limit the backswing of the rack 2140, as shown in Figure 30; The operator manually moves the pull back button in the backward direction of the rack 2140, and the stop plate responds to the pull back of the pull back button and moves downward based on the guiding effect of the chute to resist the drive claw 2130 and the stop slider 2162, thereby disengagement between the drive claw 2130 and the stop slider 2162 and the rack 2140, and thus the rack 2140 can be pulled back, as shown in Figure 31. This corresponds to the opening of the end actuator jaws.

In some embodiments, while the rack-2140 is pulled back, the trigger 2110 resets under the action of the tension spring 2300. When the trigger 2110 resets, the second operating piece 2210 moves within the button hole, and at the same time, the second operating piece 2210 moves in the opposite direction to the pressing direction under the action of the button hole, thereby resetting the second operating piece 2210; At this time, the end actuator performs the initial mode, the position of the rack 2140 is locked by the stop slider 2162, the drive claw 2130 is located at the rear side of the latch 2120, and the drive claw 2130 and the latch 2120 respectively hold the rack 2140 under the action of their respective torsion springs, Trigger 2110 is pulled by the pull spring 2300 against the convex bar of the handle housing 2190 under the action of the pull spring 2300. Pull the trigger 2110 and the end actuator starts to execute the first mode. For more details on executing the first mode, see the relevant instructions above.

In some cases, if you are not satisfied with the area of tissue squeezing and the operator does not press the mode switch button, that is, the second operating piece 2210, trigger 2110 can be operated in the opposite direction of the clamping, causing the drive claw 2130 to move backward towards the rack 2140, thereby making the latch 2120 respond to the movement of the drive claw 2130. Drive the rack 2140 to move backward, as shown in Figure 32. In some embodiments, the backward movement of rack 2140 can cause the end actuator to open, achieving the release of the tissue.

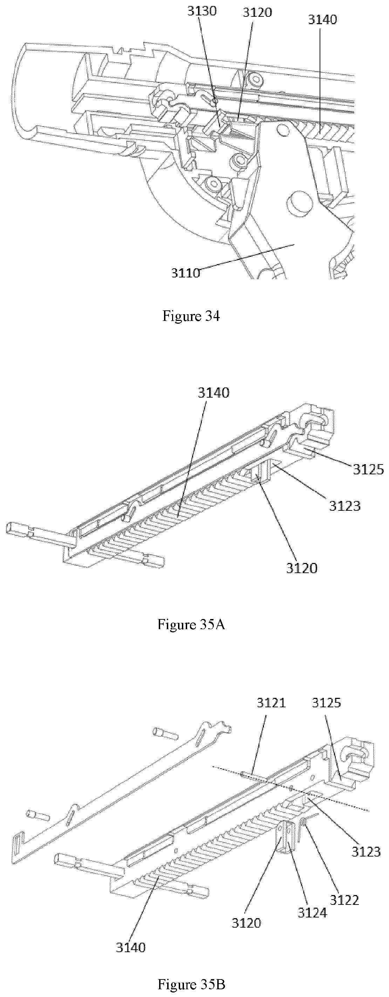

Figure 33 is a structural diagram of the drive structure 300 for surgical suturing instruments as shown in some other embodiments of this specification. Figure 34 is a partial structural diagram of the drive structure 300 for surgical suturing instruments as shown in some other embodiments of this Specification.

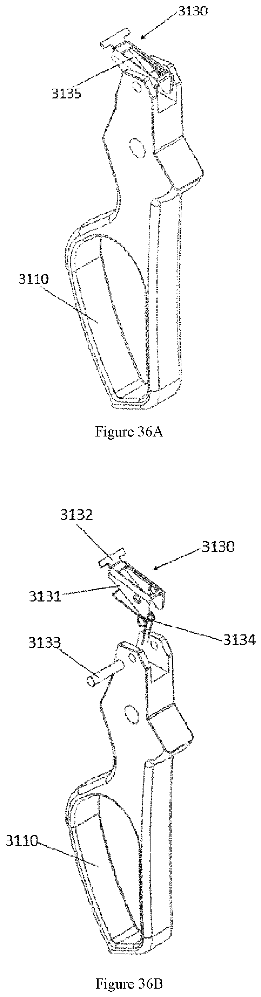

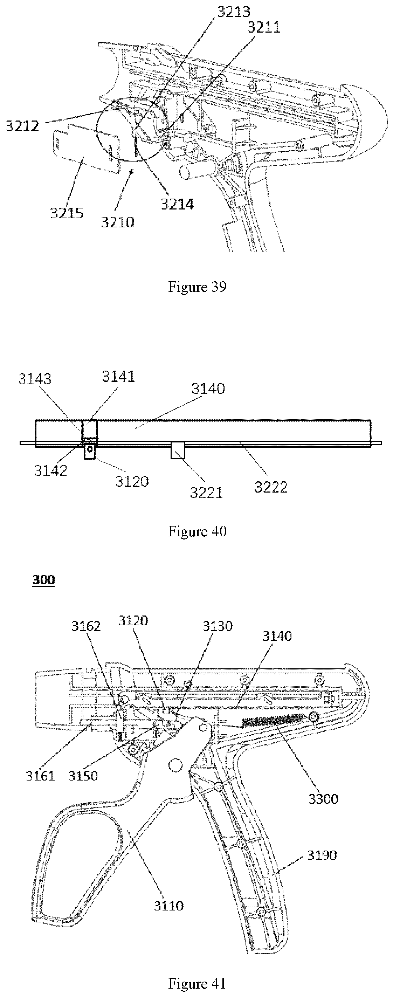

In some embodiments, as shown in Figures 33 and 34, the drive structure 300 for the surgical suture instrument includes a rack 3140, a trigger 3110, a drive claw 3130 and a mode switching mechanism. Wherein, the mode switching mechanism includes an operating component as well as a latch 3120 that is movably linked to the rack 3140. In some embodiments, the operating component may include a third operating piece 3210 (see Figure 39), which operatively moves between the third position and the fourth position to enable the switching of the surgical suturing instrument between the first mode (such as the squeezing mode) and the second mode (such as the firing mode); When the third operator 3210 moves from the third position to the fourth position, the third operator 3210 drives the drive claw 3130 to switch from the first drive mode to the second drive mode. In some embodiments, when the third operator 3210 is in the third position, the drive claw 3130 cooperates with the latch 3120, and the latch 3120 is confined within the preset travel while the drive claw 3130 is in the first drive mode. In some embodiments, the stroke range of the preset stroke is related to the extent to which the latch 3120 is restricted by the drive claw 3130. For example, the latch 3120 is restricted in the second limit slot 3135 of the drive claw 3130 (see Figure 36A), and the distance that the latch 3120 can move within the second limit slot 3135 corresponds to the preset stroke. In some embodiments, the latch 3120 is confined in the second limit slot 3135 of the drive claw 3130 (see Figure 36A), and the trigger 3110 drives the drive claw 3130 to move, thereby enabling the latch 3120 to move within the second limit slot 3135 of the drive claw 3130. This causes the rack 3140 to move forward or backward within a certain stroke. In some embodiments, in the first drive mode, the surgical suturing instrument corresponds to performing the first mode (e.g., the squeeze mode). In some embodiments, in the second drive mode, the latch 3120 disengages from the drive claw 3130, and the surgical suture instrument can perform the second mode (e.g., the firing mode).

The following will provide a detailed description of the components of the drive structure 300, and it should be noted that the following embodiments are only for illustrative illustration of the implementation of the drive structure 300 and its components.

Trigger 3110 has the same structure as trigger 110 in Drive structure 100 and will not be repeated.

In some embodiments, the latch 3120 can be movably connected to the rack 3140 to limit the movement of the rack 3140. For example, the latch 3120 can be used in conjunction with other structures (such as the second limit slot 3135) to lock the rack 3140, thereby restricting the forward or backward movement of the rack 3140; The latch 3120 can also remove the restriction on the movement of the rack 3140 by disengaging from other structures, such as the second limit slot 3135. For information on how the latch 3120 connects to the rack 3140, see Figures 35A and 35B and their related descriptions. For details on limiting the movement of the rack 3140 through the coordination of the latch 3120 with the second limit slot 3135, see the relevant description below.

Figure 35A is a schematic diagram of the structure of the latch 3120 as shown in some other embodiments of this specification. Figure 35B is an explosion diagram of the latch 3120 as shown in some other embodiments of the present Specification.

In some embodiments, as shown in Figures 35A and 35B, the latch 3120 can take on various shapes, for example, cuboid, triangular, cylindrical, etc. In some embodiments, a slot 3123 is provided below the rack 3140 (see arrow g direction in Figure 33), and the latch 3120 can be movably set in slot 3123 of the rack 3140, for example, the latch 3120 can be set in slot 3123 by means of a snap-fit connection, a pivot connection, or any other viable connection. In such a way that the latch 3120 should be able to be fully or partially inserted into slot 3123, or protrude downward relative to the rack 3140. In some embodiments, when the latch 3120 protrudes downward relative to the rack 3140, the downward protruding part of the latch 3120 can be coordinated with other components (the second limit slot 3135 as shown in Figure 36A) to limit the movement of the rack 3140.

In some embodiments, the latch 3120 May be rotatable with the rack 3140, and the latch 3120 is able to rotate relative to the connection so that the latch 3120 can be rotated from being fully or partially placed in slot 3123 to protruding downward relative to the rack 3140, or by protruding downward relative to the rack 3140, Rotate to be fully inserted into slot 3123.

In some embodiments, as shown in Figure 35B, the latch 3120 can be set at the far end of the rack 3140 (i.e., the side close to the forward direction of the rack 3140, the forward direction as indicated by the arrow h in Figure 33), and the latch 3120 and the rack 3140 can be armature connected through the first pivot 3121. The first pivot 3121 May be provided with the first torsion spring 3122, through which the latch 3120 can be elastically pressed against the rack 3140, so that the latch 3120 remains in a state of protruding downward relative to the rack 3140 without external force, which is the initial position of the latch 3120; When the latch 3120 rotates around the center of the first torsion spring 3122 (i.e., the first pivot 3121), the first torsion spring 3122 has the rotational force to return the latch 3120 to its original position.

In some embodiments, the latch 3120 is provided with a slot 3124 for setting the first torsion spring 3122 so that a large part of the first torsion spring 3122 (such as the part set within the slot 3124) can occupy the same space as the latch 3120 itself, saving space and making the device structure compact.

In some embodiments, the drive claw 3130 can be movably connected to the trigger 3110, and the operator can transmit power to the drive claw 3130 by pulling the trigger 3110, and then the drive claw 3130 transmits power to the rack 3140 to drive the rack 3140 to move.

Figure 36A is a schematic diagram of the structure of the drive claw 3130 as shown in some other embodiments of this specification. Figure 36B is an explosion diagram of the drive claw 3130 as shown in some other embodiments of the present Specification.

In some embodiments, the drive claw 3130 is provided with a second limit slot 3135 (see Figure 36A), and the latch 3120 can cooperate with the drive claw 3130 when the latch 3120 is located in the second limit slot 3135. In some embodiments, the length of the second limit slot 3135 can be set as needed, for example, the length of the second limit slot 3135 can be 10mm-20mm, etc. In some embodiments, the drive claw 3130 has a first drive mode and a second drive mode. For example only, in the first drive mode, the latch 3120 in the downward protruding state relative to the rack 3140 can be clamped into the second limit slot 3135 of the drive claw 3130, thus the range of motion of the drive claw 3130 driven by the trigger 3110 and the range within the slot of the second limit slot 3135 correspond to the range of motion of the latch 3120. This allows the rack 3140 to move forward or backward within a certain range. In the second drive mode, when the latch 3120 is outside the second limit slot 3135, the latch 3120 disengages from the drive claw 3130, that is, the latch 3120 separates from the drive claw 3130 and is no longer restricted by the position of the drive claw 3130. In some embodiments, the first drive mode of the drive claw 3130 corresponds to the end actuator of the surgical suturing instrument performing the first mode (e.g., squeezing mode), and the second drive mode of the drive claw 3130 corresponds to the end actuator of the surgical suturing instrument performing the second mode (e.g., firing mode). In some embodiments, the first mode and the second mode can be switched by manual or automatic means. Surgical suturing instruments, for example, may include a third operating piece 3210 that can be manually operated by the operator (see Figure 39), by manually adjusting the drive mode of the drive claw 3130 to switch the end actuator of the surgical suturing instrument to either the first mode or the second mode. More on mode switching can be found in the relevant description below.

In some embodiments, as shown in Figures 36A and 36B, the driving claw 3130 May include the main body 3131 and the end of the claw 3132. The materials and respective functions of the end of the claw 3132 and the main body 3131 are the same as those of the main body 131 and the end of the claw 132 in the driving structure 100, and will not be elaborated further.

In some embodiments, the drive claw 3130 can be movably connected to the trigger 3110, for example, the drive claw 3130 can be rotatably connected to the trigger 3110.

In some embodiments, as shown in Figure 36B, the drive claw 3130 can be set on the side of the trigger 3110 close to the rack 3140, and the trigger 3110 can be pivotly connected to the drive claw 3130 through the second pivot 3133, which is threaded with the second torsion spring 3134 when the trigger 3110 is pulled. The trigger 3110 can rotate around the center of the second torsion spring 3134 (i.e., the second pivot 3133), and the second torsion spring 3134 has the rotational force to return the trigger 3110 to its original position, thus the relative position of the trigger 3110 and the drive claw 3130 can be elastically limited by the second torsion spring 3134. It should be noted that the second torsion spring 3134 is not a necessary structure, and even without the second torsion spring 3134, the trigger 3110 can return to its original position by other means, for example, by manual return.

In some embodiments, the rack 3140 can be configured to be able to move forward in the direction of the arrow h as shown in Figure 33.

In some embodiments, multiple teeth may be provided on the rack 3140, and the coordination of the drive claw 3130 with the teeth can push the rack 3140 forward. The fit between the drive claw 3130 and the rack 3140 is the same as that between the drive claw 130 and the rack 140 in the drive structure 100 and will not be elaborated further.

In some embodiments, a rack step 3125 May be provided on the rack 3140 (see Figures 35A and 35B), and the drive claw 3130 can cooperate with the rack step 3125 to push the rack 3140 forward. In some embodiments, the rack step 3125 can be set at the far end of the rack 3140, and the latch 3120 can be set between the teeth of the rack 3140 and the rack step, making full use of the empty space on the rack 3140 where the teeth have not yet been distributed, resulting in a compact device structure.

In some embodiments, the drive structure 300 May also include the tension spring 3300 (see Figure 33), the tension spring 3300 has the same structure and function as the tension spring 1300 in the drive structure 100, which will not be elaborated further.

In some embodiments, as shown in Figure 33, the drive structure 300 May also include a forward slider 3161 and a stop slider 3162. The forward sliders 3161 and the anti-retreat sliders 3162 have the same structure and function as the forward sliders 161 and the anti-retreat sliders 162 in the drive structure 100, and will not be elaborated further.

In some embodiments, the drive structure 300 May also include the connecting rod 3150 (see Figure 33), the connecting rod 3150 has the same structure and function as the connecting rod 150 in the drive structure 100, which will not be elaborated further.

The third operating piece 3210 can be operated by the operator, thereby achieving functions such as mode switching of surgical suture instruments. In some embodiments, the third operating piece 3210 can be operated between the third position and the fourth position. In some embodiments, when the third operating piece 3210 is in the third position, the drive claw 3130 cooperates with the latch 3120, that is, when the latch 3120 is in the second limit slot 3135 of the drive claw 2130, the latch 3120 is confined within the preset travel and the drive claw 3130 is in the first drive mode; When the third operating piece 3210 is in the fourth position, that is, when the latch 3120 is outside the second limit slot 3135 of the drive claw 3130, the latch 3120 disengages from the drive claw 3130, and the drive claw 3130 is in the second drive mode. In some embodiments, the first drive mode can correspond to the end actuator of the surgical suturing instrument performing the first mode, which can be the squeezing mode, where the tool component of the end actuator, such as the jaws, performs a closing operation to squeeze the tissue; The second drive mode can correspond to the end actuator of the surgical suturing instrument performing the first mode, and the second mode can be the firing mode, that is, the tool component of the end actuator, such as the cutter and the pinning device, etc., performing the forward direction movement, performing the cutting and suturing operation on the tissue. For more information on operating the 3210 movement of the third operating piece to achieve mode switching of the surgical suturing instrument, see Figures 39-53 and their related descriptions, which will not be repeated here.

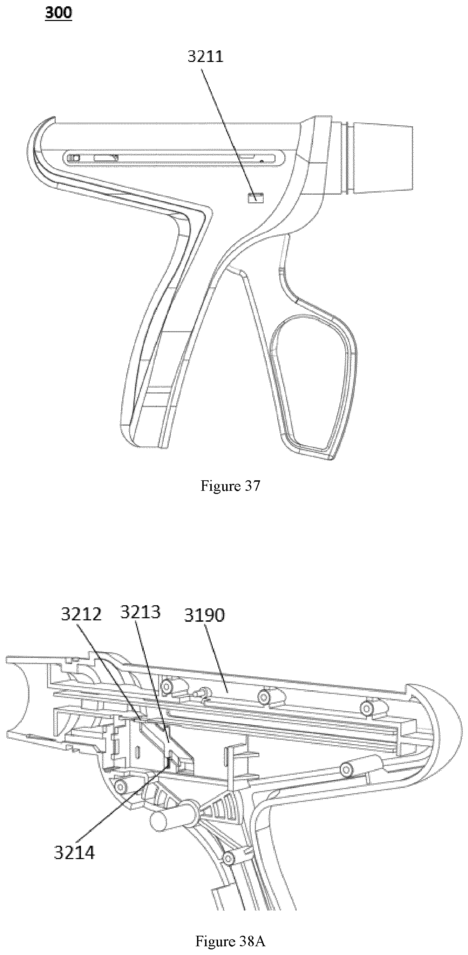

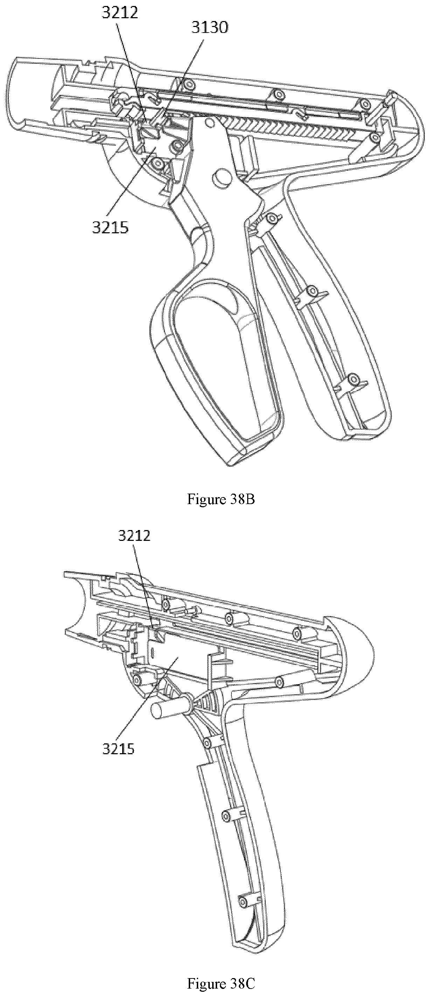

Figure 37 is a schematic diagram of the operating end of the third operating device 3210 as shown in some other embodiments of this specification. Figure 38A is a structural diagram of the third operating device 3210 as shown according to some embodiments of the present Specification. Figure 38B is a structural diagram of the third operating device 3210 as shown according to some other embodiments of the present Specification. Figure 38C is a structural diagram of the third operating device 3210 as shown according to some other embodiments of the present Specification. Figure 39 is an explosion diagram of the third operating device 3210 as shown according to some other embodiments of this specification.

In some embodiments, the third operating piece 3210 May include an operating end 3211 (see Figure 37) and a contact end 3212 (see Figures 38A, 38B, and 38C), with the operating end 3211 being operable to move, thereby driving the contact end 3212 to move up and down, and the contact end 3212 being in contact with the driving claw 3130. It is used to drive the drive claw 3130 to move. In some embodiments, the operation end 3211 May be relative to the exposure outside the handle housing 3190, and the operator can directly touch the operation end 3211 to operate; The operation end 3211 May also be hidden within the handle housing 3190 but accessible to the operator, for example, by tool contact, etc.

In some embodiments, the third operating piece 3210 can be in contact with the drive claw 3130. By operating on the third operating piece 3210, the drive claw 3130 can be driven to move, changing the relative position state of the drive claw 3130 to the latch 3120, thereby changing the operating mode of the drive structure 300, that is, the working mode corresponding to switching the suturing instrument. In the example, by moving the third operating piece 3210 downward, it can drive the driving claw 3130 downward, causing the latch 3120 located in the second limit slot 3135 of the driving claw 3130 to disengage from the second limit slot 3135, even if the suturing instrument is switched from mode 1 to Mode 2. For more on mode switching, see the relevant description below.

In some embodiments, the movement of the third operant 3210 between the third position and the fourth position can be either linear or rotational. In some embodiments, the third operating piece 3210 includes an operating end 3211 that can be operated by the operator, as well as a contact end 3212 for contact with the drive claw 3130 to drive the movement of the drive claw 3130. In some embodiments, the movement mode of the operating end 3211 May be the same as or different from that of the contact end 3212. For example, both the operation end 3211 and the contact end 3212 can move in a straight line. For example, the operation end 3211 can perform rotational motion, and the contact end 3212 can perform linear motion. In some embodiments, as an example, as shown in Figure 38A, the third operating piece 3210 May include a linear motion piece 3213 that can move up and down. The linear motion piece 3213 includes an operating end 3211 that protrudes outward towards the handle housing 3190 and a contact end 3212 that protrudes inwardly towards the handle housing 3190. In some embodiments, the operating end 3211 is operated by the operator to move up and down, which can drive the contact end 3212 to move up and down through the linear motion piece 3213, and since the contact end 3212 is in contact with the drive claw 3130, it can drive the drive claw 3130 to move up or down accordingly. For example, the linear motion 3213 drives the contact end 3212 to move downward, which in turn drives the drive claw 3130 to move downward, causing the latch 3120 to disengage from the second limit slot 3135 of the drive claw 3130, thereby enabling the drive claw 3130 to switch from the first drive mode to the second drive mode.

In some embodiments, as shown in Figure 39, the third operating piece 3210 can be set in the mounting slot inside the handle housing 3190, which is the space used to accommodate the third operating piece 3210. In some embodiments, the drive structure 300 May also include a baffle 3215, which can cover the mounting slot to limit the mounting position of the third operating piece 3210, so that the third operating piece 3210 can only move up and down in the mounting slot and not sideways.

In some embodiments, the bottom of the third operating piece 3210 can be elastically connected to the bottom of the mounting slot, allowing the third operating piece 3210 to return from the fourth position to the third position without external force. For example, the bottom of the third operating element 3210 can be connected to the bottom of the mounting slot by a spring 3214, which can be used to apply an elastic force to the third operating element 3210, allowing it to return from the fourth position to the second position. In some embodiments, as shown in Figure 38A, the spring 3214 can be respectively connected to the bottom of the mounting slot and the bottom of the linear motion piece 3213. Under the elastic force, the linear motion piece 3213 is located at the uppermost position of its upward movement, that is, the third position of the third operating piece 3210, and the operating end 3211 can be pressed down by the operator. Until the linear motion piece 3213 moves to the lowest position where it can move downward, that is, the fourth position of the third operating piece 3210, during the movement from the third position to the fourth position, the linear motion piece 3213 drives the contact end 3212 to move downward and drives the drive claw 3130 to move downward. After the operator stops applying downward pressure to the operating end 3211, the linear motion piece 3213 is lifted back to the third position by the elastic force of the spring 3214.

It is understandable that the third operating piece 3210 can be set in drive structure 100 instead of the aforementioned first operating piece 210, or in drive structure 200 instead of the aforementioned second operating piece 2210. In the example, the operator can control the drive claw 130 to switch from the first drive mode to the second drive mode by pressing down the operation end 3211 of the third operate 3210, which can drive the limit part 1364 to move downward and detach from the first limit slot 121. By the same token, the aforementioned first operator 210 or second operator 2210 can replace the third operator 3210 and be set in the drive structure 300. In the example, when the first operator 210 is pressed and moves from the initial position to the pressed position, the first operator 210 can drive the drive claw 3130 to move downward, thereby causing the latch 3120 to disengage from the second limit slot 3135, thereby controlling the drive claw 3130 to switch from the first drive mode to the second drive mode.

In some embodiments, the manipulator can be set on the trigger 110, and because it is closer to the position where the operator holds it, the operator can easily perform one-handed operation. For example, the first operating piece 210 (or the second operating piece 2210) is set on the trigger 110, and the operator can easily switch modes by pressing the first operating piece 210 (or the second operating piece 2210). The operator can operate with one hand, that is, press while holding the surgical suturing instrument, and the operation is very convenient. In some embodiments, the operating piece can be set on the handle housing 190, and the operator can switch modes through the operating piece on the handle housing 190. For example, the third operator 3210 is set on the handle housing 3190, and the operator can switch the mode by towing the third operator 3210. Understandably, the operator can be set as needed, as long as it is easy for the operator to operate.

In some embodiments, the latch 3120 can be set to move upwards, and the operator can disengage the latch 3120 from the drive claw 3130 by moving the latch 3120 upwards.

Figure 40 is a schematic diagram of the structure of the latch 3120 and the rack 3140 as shown in some other embodiments of this specification.

In some embodiments, as shown in Figure 40, a slide groove 3141 May be provided on the side of the rack 3140, the extension direction of the slide groove 3141 is perpendicular to the forward direction of the rack 3140, a slidably arranged slider 3142 is provided in the slide groove 3141, the slider 3142 is rotatably connected to the latch 3120, and a protrusion 3143 is protruded on the slider. The protrusion 3143 protrudes towards the side of the rack 3140. By moving the protrusion 3143 upwards, it can drive the slider 3142 and the latch 3120 upwards.

In some embodiments, the operating component may include a fourth operating component that can move up and down. The fourth operating component includes a toggle 3221 located outside the handle housing 3190 and a strip 3222 located inside the handle housing 3190, with the strip 3222 fixedly connected to the toggle 3221. The strip 3222 is located on the side of the rack 3140 and beneath the protrusion 3143. The operator can move the puller 3143 upward by pulling the puller 3221 upward, thereby moving the slider 3142 and the latch 3120 attached to it upward, causing the latch 3120 to disengage from the drive claw 3130, thus switching the drive claw 3130 from the first drive mode to the second drive mode.

In some embodiments, the strip 3222 extends along the forward direction of the rack 3140, and the length of the strip 3222 can be set as needed. Since the forward or backward movement of the rack 3140 drives the chute 3141 and the sliders 3142 within it forward or backward, the length of the strip 3222 can be matched with the extent to which the chute 3141 moves along with the rack 3140, thus allowing the third operating piece to easily pull the protrusion 3143.

In some embodiments, the inner side of the chute 3141 May have a layer structure with greater friction, for example, a silicone pad on the inner side of the chute 3141. Under the effect of friction, the sliding block can resist gravity, allowing the sliding block 3142 to maintain its position within the slot 3141 without any additional force (i.e., the force applied by the operator through the fourth operating piece), and it will not slide down due to gravity.

In some other embodiments, without the need for an additional fourth operating piece, the handle housing 3190 May be provided with a long groove that extends along the forward direction of the rack 3140, and the protrusion 3143 extends out of the handle housing 3190 through the long groove, allowing the operator to pull the protrusion 3143 upwards. The length of the long slot can be matched with the extent to which the slide slot 3141 moves along the rack 3140, and the width of the long slot can be matched with the length of the slide slot 3141, so that the operator can easily pull the convex slot 3143.

It is understandable that the fourth operating element and its related structure as shown in Figure 40 can replace the third operating element 3210 and be set in the drive structure 300, can replace the second operating piece 2210 and be set in the drive structure 200, or can replace the first operating piece 210 and be set in the drive structure 100.

In some embodiments, as shown in Figure 33, the drive structure 300 May also include a pull-back structure, which may include a pull-back button 3180 and a stop plate 3170. The structure and function of the pull-back structure are the same as those of the pull-back structure in drive structure 100 and will not be elaborated here.

Some embodiments of this specification provide a surgical suture instrument which may include a handle unit, an end actuator, and a drive structure 300 of any of the above embodiments. The drive structure 300 May be arranged in the handle housing 3190 of the handle unit, the trigger 3110 can be rotated and arranged on the handle housing 3190, and the rack 3140 can be slid and arranged on the handle housing 3190. The end actuator can be connected to the drive structure 300, for example, the cutter and pinning device of the end actuator can be connected to the rack 3140. In some embodiments, the end actuator has multiple working modes, and different working modes can be controlled and executed through the drive structure 300 based on the operation of the handle unit, and the operation of the handle unit can also be adjusted through the drive structure 300 to adjust the working mode of the end actuator. More information on adjusting the working mode of the end actuator through the drive structure can be found in the relevant description below.

In some embodiments, the end actuator has multiple executable working modes, and the operator can select the appropriate mode based on clinical needs. In some embodiments, surgical suturing devices may have initial mode, first mode, and second mode. The initial mode may correspond to the initial mode of the surgical suturing instrument, which corresponds to the original state of the surgical suturing instrument in which no work has been performed. The first mode corresponds to the compression mode of the surgical suturing instrument. Pressing the trigger 3110 corresponds to the gradual closure of the jaws of the end actuator, clamping and further squeezing of the tissue between the jaws. In this mode, pressing the trigger 3110 can be used to achieve tissue compression. Drive structure 300 does not need to drive other tool components (such as cutters, etc.) forward to perform operations such as cutting. The second mode can correspond to the firing mode of surgical suturing instruments, which can include push nail forming and push knife cutting, etc. The drive structure 300 needs to drive related components such as rack 3140 forward, so that the tool components of the end actuator connected to rack 3140, such as the cutter and the pinning device, perform operations such as cutting and suturing at the same time. The following illustrates the working mode of the surgical suture instrument through some embodiments. It should be noted that the following is only an example and not a limitation to this specification. The surgical suture instrument and its drive structure 300 in the embodiments of this specification can be used in any other feasible way of use.

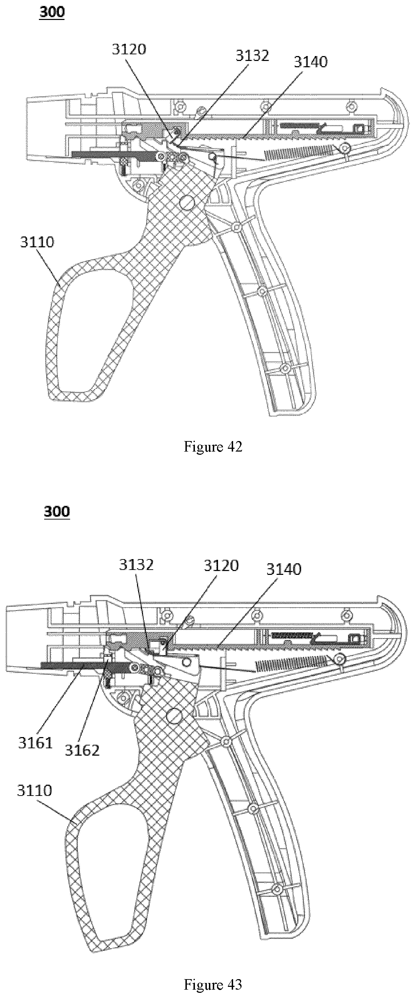

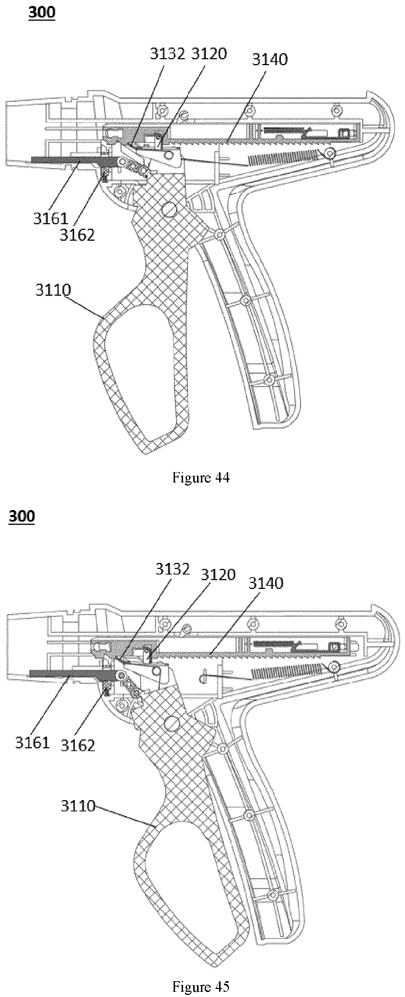

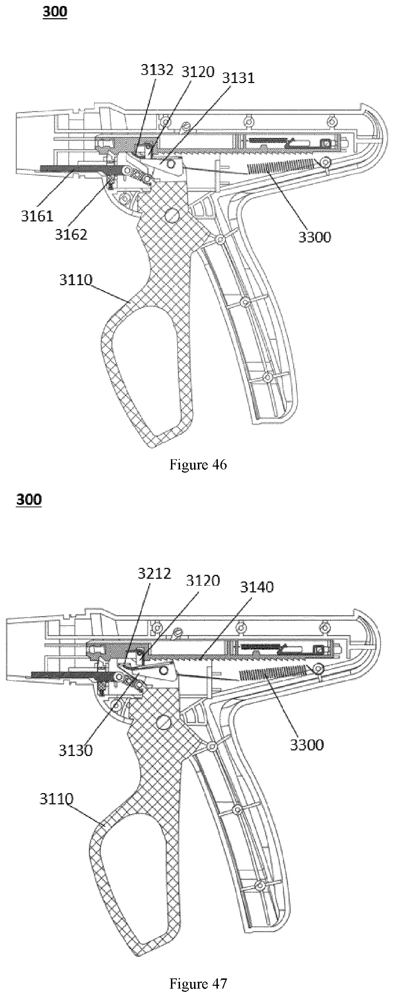

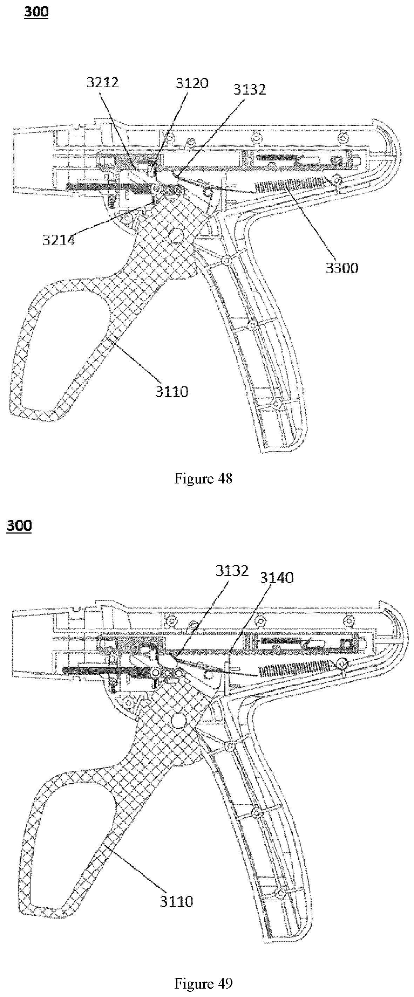

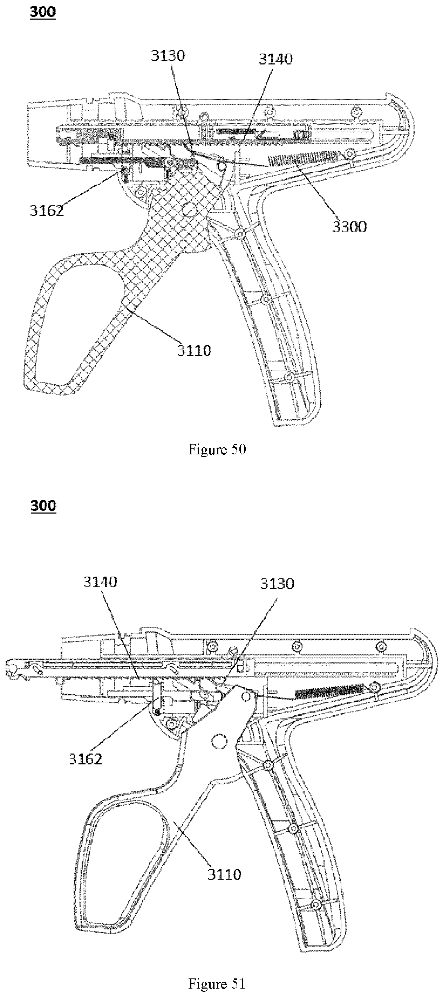

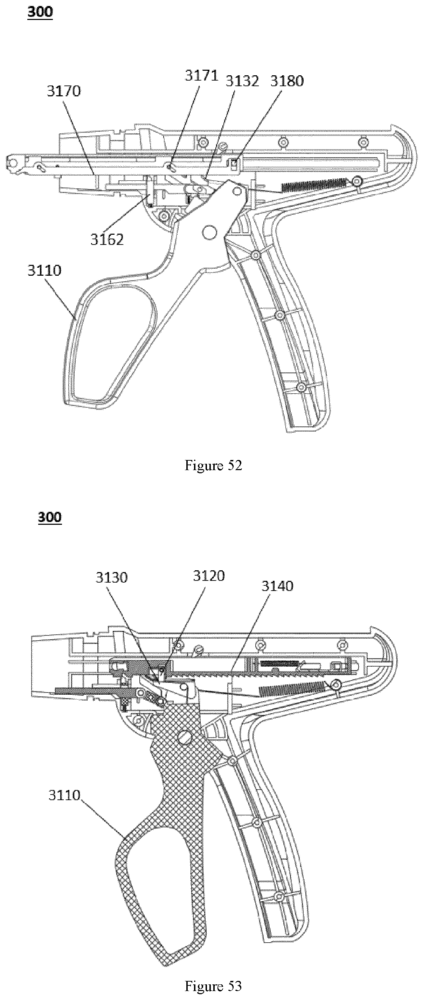

Figure 41 is a schematic diagram of the initial mode as shown in some embodiments of this specification. Figure 42 is a schematic diagram of the first pattern as shown according to some other embodiments of this specification. Figure 43 is a schematic diagram of the first pattern as shown according to some other embodiments of this specification. Figure 44 is a schematic diagram of the first pattern as shown according to some other embodiments of this specification. Figure 45 is a schematic diagram of the first pattern as shown according to some other embodiments of this specification. Figure 46 is a schematic diagram of the mode switching as shown according to some other embodiments of this specification. Figure 47 is a schematic diagram of the mode switching as shown according to some other embodiments of this specification. Figure 48 is a schematic diagram of the mode switching as shown according to some other embodiments of this specification. Figure 49 is a schematic diagram of the second mode as shown according to some other embodiments of this specification. Figure 50 is a schematic diagram of the second pattern as shown according to some other embodiments of this specification. Figure 51 is a schematic diagram of the second pattern as shown according to some other embodiments of this specification. Figure 52 is a schematic diagram of the second pattern as shown according to some other embodiments of this specification. Figure 53 is a schematic diagram of the tissue release operation as shown in some other embodiments of this specification.

As shown in Figure 41, in some embodiments, the position of the rack 3140 that drives structure 300 is locked when the end actuator performs the initial mode. In the example, the anti-retreat slider 3162 is lifted by the elasticity of the spring and holds the rack 3140 against it on the forward path of the rack 3140, restricting the advance of the rack 3140; The drive claw 3130 is located at the rear side of the latch 3120, and the drive claw 3130 and the latch 3120 respectively hold the rack 3140 under the action of their respective torsion springs; The third operating piece 3210 is lifted by the elastic effect of spring 3214; The trigger 3110 can be applied to the rearward direction of the rack 3140 under the action of the pull spring 3300, and the handle housing 3190 can be set with the corresponding convex bar, and the trigger 3110 can be pulled by the pull spring 3300 against the convex bar.

In some embodiments, the movement of the rack 3140 that drives the structure 300 is restricted when the end actuator performs the first mode. In the example, when the trigger 3110 is pulled, the trigger 3110 drives the drive claw 3130 forward, and the end of the claw of the drive claw 3130, 3132, pushes the latch 3120 to rotate, as shown in Figure 42; As shown in Figure 43, when the trigger 3110 is engaged to the set position, the end of the claw of the drive claw 3130, 3132, disengages from the latch 3120, and the latch 3120 returns to its original position under the action of the first torsion spring 3122, then the latch 3120 bounces into the second limit slot 3135 of the drive claw 3130; Continuing to pull the trigger 3110, the stop slider 3162 is pushed down by the protrusion of the forward slider 3161 and begins to release the limit on the rack 3140; As shown in Figure 44, continue to pull the trigger 3110, the stop slider 3162 is gradually pushed down, the drive claw 3130 is driven forward by the trigger 3110, and the end of the claw of the drive claw 3130, 3132, touches the rack step of the rack 3140. At this point, the stop slider 3162 has been fully pushed down, completely lifting the limit on the rack 3140; As shown in Figure 45, when the trigger 3110 is pressed again, the rack 3140 advances under the push of the end of the drive claw 3130's claw 3132, thereby enabling the end actuator of the surgical suture instrument to perform the clamping opening closure operation to complete the squeezing action on the tissue. Since the latch 3120 remains within the second limit slot 3135 of the drive claw 3130 throughout the process and does not disengage, the movement of the rack 3140 is controlled by the movement of the trigger 3110. Based on the trigger 3110 driving the drive claw 3130 to move, the rack 3140 can move forward and backward within a certain range. This enables the closing and opening of the partial actuator.

In some embodiments, the operator can switch the mode of the surgical suture instrument through the third operating piece 3210, that is, from the first mode to the second mode. In the example, the operator can release the trigger 3110, which automatically returns to the initial position under the action of the pull spring 3300, and eventually the trigger 3110 and the drive claw 3130 are limited by the latch 3120, as shown in Figure 46; By pressing the operating end 3211 of the third operating piece 3210, the contact end 3212 of the third operating piece 3210 comes into contact with the driving claw 3130 to achieve a counterclockwise rotation of the driving claw 3130, allowing the latch 3120 to disengage from the second limit slot 3135 of the driving claw 3130. When the driving claw 3130 is completely disengaged from the latch 3120, At this point, no structure limits the drive claw 3130, as shown in Figure 47, the drive claw 3130 will, together with the trigger 3110, move in the backward direction of the rack 3140 under the action of the pull spring 3300 to the rear side of the latch 3120; The drive claw 3130 will be under the action of the second torsion spring 3134, with the tip of the claw 3132 against the teeth of the rack 3140; The operator releases the operating end 3211 of the third operating piece 3210, and the linear motion piece 3213 of the third operating piece 3210 is lifted back to its initial position under the action of the spring 3214, as shown in Figure 48.

In some embodiments, when the end actuator performs the second mode, the rack 3140 of the drive structure 300 is able to move forward. In the example, the end actuator performs the second mode when the claw end 3132 of the drive claw 3130 holds against the teeth of the rack 3140; The operator pulls the trigger 3110 and pushes the rack 3140 through the drive claw 3130 to advance the rack 3140, as shown in Figure 49. In some embodiments, the tool component of the surgical suturing instrument connected to the rack 3140, for example, the cutter and the pinning device, can achieve cutting and suturing of the tissue based on the advance of the rack 3140; The operator releases the trigger 3110, and the drive claw 3130, together with the trigger 3110, can retreat under the action of the pull spring 3300, and the anti-retreat slider 3162 is bounced up under the action of the spring element to hold against the teeth of the rack 3140, thereby limiting the retreat of the rack 3140, as shown in Figure 50; When the rack 3140 has completed the full stroke (corresponding to after the user has finished sewing), the drive claw 3130 and the anti-retreat slider 3162 can hold against the teeth of the rack 3140, as shown in Figure 51; The operator manually moves the pull back knob 3180 in the backward direction of the rack 3140, and the stop plate 3170 responds to the pull back of the pull back knob and moves downward based on the guiding effect of the chinion to resist the drive claw 3130 and the stop slider 3162, thereby disengaging the drive claw 3130 and the stop slider 3162 with the rack 3140. As a result, the rack 3140 can be pulled back, as shown in Figure 52, corresponding to the opening of the end actuator jaws.

In some cases, if you are not satisfied with the area where the tissue is being squeezed and the operator does not press the mode switch button, the trigger 3110 can be operated in the opposite direction of the crimping, causing the drive claw 3130 to move backward towards the rack 3140, so that the latch 3120 responds to the movement of the drive claw 3130 and drives the rack 3140 to move backward. Figure 53 shows this. In some embodiments, the backward movement of rack 3140 can cause the end actuator to open, achieving the release of the tissue.

The basic concepts have been described above. Clearly, the above detailed disclosures are only for example and do not constitute a limitation to the present specification for those skilled in the art. Although not expressly stated here, those skilled in the art may make various modifications, improvements and corrections to this specification. Such modifications, improvements, and amendments are recommended in this specification and therefore remain in the spirit and scope of the model embodiments of this Specification.

## Claims

1. A drive structure for a surgical suturing instrument, wherein the drive structure is arranged in a handle housing (190) of the handle part of the surgical suturing instrument, and the drive structure includes:
A rack (140) that can move linearly is arranged in the handle housing (190);
A trigger (110) is rotatably arranged on the handle housing (190);
Drive assembly, the drive assembly comprises a drive claw (130) that is movably connected to the trigger (110);
Mode switching mechanism, including an operating component and a latch (120) that is movably connected to the rack (140); The operating component is connected to the driving component; The operation component controls whether the drive component fits with the latch (120), thereby controlling the drive claw (130) to switch between the first drive mode and the second drive mode.

2. The drive structure of claim 1, wherein the operation component comprises an operable switching slider (136), and the drive component comprises a limit part (1364) fixedly connected to the switching slider (136); The switching slider (136) drives the limit part (1364) to move between the first position and the second position; When the limit unit (1364) is at the first position, the limit unit (1364) is in the state of being restricted within the preset stroke by the latch (120), and the drive claw (130) is in the first drive mode; When the limit part (1364) is at the second position, the limit part (1364) is in the state of being unrestricted from the latch (120), and the drive claw (130) is in the second drive mode.

3. The drive structure of claim 2, wherein the latch (120) and the rack (140) are rotatably connected by a rotating shaft member (123);
The rack (140) has a stop part (142), which is in contact with the latch (120) to limit the angle at which the latch (120) rotates towards the proximal end of the rack (140).

4. The drive structure of claim 3, wherein the rack (140) comprises a first limit slot (121), and the latch (120) can be rotatingly set at the near-end of the first limit slot (121), when the limit part (1364) is located in the first limit slot (121), The far-end wall of the first limit slot (121) restricts the forward movement of the limit part (1364), and the far-end wall of the latch (120) restricts the backward movement of the limit part (1364).

5. The drive structure of claim 4, wherein the rack (140) is provided with a avoidance slot (141) along the extension direction of the rack (140), the avoidance slot (141) is arranged on the side of the rack (140) close to the drive claw (130) and on the side of the near-end of the first limit slot (121), and connecting with the first limit slot (121), the avoidance slot (141) is capable of accommodating the limit part (1364).

6. The drive structure of claim 4, wherein the latch (120) comprises an elastic reset element, and the latch (120) is connected to the rack (140) through the elastic reset element.

7. The drive structure of claim 6, wherein the elastic reset element comprises a tension spring element (122), one end of the tension spring element (122) is attached to the rack (140), and the other end of the tension spring element (122) is attached to the latch (120).

8. The drive structure of claim 1, wherein the rack (140) is provided with rack steps and multiple teeth; The drive claws (130) push the rack (140) forward by cooperating with the rack steps or the teeth.

9. The drive structure of claim 2, wherein the drive claw (130) comprises a main body part (131) and the end of the claw part (132), the main body part (131) is fixedly connected to the end of the claw part (132), and the main body part (131) is rotatably connected to the trigger (110),the main body (131) is provided with an opening for the limit part (1364) to pass through, and the end of the claw (132) is used to push the rack (140).

10. The drive structure of claim 2, wherein the operating assembly further includes a first operating piece (210), which is pressably arranged on the trigger (110), and the switching slider (136) is Transmittingly connectedto the first operating piece (210). The first operating piece (210) moves between the initial position and the pressed position. When the first operating piece (210) is pressed and moves from the initial position to the pressed position, the first operating piece (210) drives the switching slider (136) to move the limit part (1364) from the first position to the second position.

11. The drive structure of claim 10, wherein the first operating piece (210) comprises a first inclined plane (211), and the switching slider (136) comprises a second inclined plane (1361) that cooperates with the first inclined plane (211); When the first operating piece (210) is pressed in the first direction and moves from the initial position to the pressed position, the second inclined plane (1361) moves along the first inclined plane (211) to drive the switching slider (136) to move from a position close to the rack (140) to a position away from the rack (140);
The first operating piece (210) includes a third inclined plane (213); The switching slider (136) includes a fourth inclined plane (1362) that cooperates with the third inclined plane (213); When the first operating piece (210) is pressed in the second direction and moves from the initial position to the pressed position, the fourth inclined plane (1362) moves along the third inclined plane (213), to drive the switching slider (136) to move from a position close to the rack (140) to a position away from the rack (140);
The first direction is opposite to the second direction.

12. The drive structure of claim 11, wherein the first operating piece (210) comprises a locking slot, and the switching slider (136) comprises a locking structure cooperating with the locking slot; The relative motion of the first operating piece (210) and the switching slider (136) is restricted when the first operating piece (210) is pressed and moves to the locking structure cooperating with the locking slot.

13. The drive structure of claim 10, wherein the drive structure further comprises a button hole (220) arranged on the handle housing (190), and the first operating piece (210) is slidably arranged in the button hole (220), The button hole (220) comprises a button reset part (221) that cooperates with the first operating piece (210), and the button reset part (221) is used to apply a force to the first operating piece (210) in a direction opposite to the pressing direction; When the trigger (110) is reset, the first operating piece (210) slides within the button hole (220), and the first operating piece (210) moves in the opposite direction to the pressing under the action of the button reset part (221).

14. The drive structure of claim 10, wherein the trigger (110) is provided with a first accommodation slot, the switching slider (136) is provided in the first accommodation slot, and a first elastic member (134) is provided between the switching slider (136) and the bottom of the first accommodation slot.

15. The drive structure of claim 1, wherein it further comprises a forward slider (161) and a stop slider (162), one end of the trigger (110) is movably connected to the forward slider (161), and the stop slider (162) is used to restrict the movement of the rack (140); The trigger (110) drives the anti-reverse slider (162) to move by moving the forward slider (161) in the forward direction of the rack (140), thereby relieving the restriction of the anti-reverse slider (162) on the movement of the rack (140).

16. The drive structure of claim 15, wherein it further comprises a connecting rod (150), one end of which is rotatably connected to the forward slider (161), and the other end of the connecting rod (150) is rotatably connected to the trigger (110).

17. The drive structure of claim 1, wherein it further comprises a tension spring (300), one end of which is connected to the handle housing (190) and the other end is connected to the trigger (110) for the purpose of applying force from the trigger (110) to the retractable direction of the rack (140).

18. The drive structure of claim 1, wherein the operating component comprises a second operating piece (2210), which can be pressed and arranged on the trigger (2110), and which moves between the initial position and the pressed position, When the second operating piece (2210) is pressed and moves from the initial position to the pressed position, the second operating piece (2210) drives the driving claw (2130) to switch from the first driving mode to the second driving mode; In the first drive mode, the drive claw (2130) cooperates with the latch (2120), and the latch (2120) is confined within the preset travel; In the second drive mode, the latch (2120) disengages from the drive claw (2130).

19. The drive structure of claim 18, wherein the drive assembly comprises a drive slider (2136) which is transmitted and connected between the drive claw (2130) and the second operating piece (2210), and the drive slider (2136) is arranged on the trigger (2110), When the second operating piece (2210) is pressed and moves from the initial position to the pressed position, the second operating piece (2210) drives the drive slider (2136) to move from a position close to the rack (2140) to a position far away from the rack (2140). The drive slider (2136) drives the drive claw (2130) to switch from the first drive mode to the second drive mode.

20. The drive structure of claim 19, wherein a second accommodation groove is provided on the trigger (2110), the drive slider (2136) is arranged in the second accommodation groove, and a second elastic member (2134) is provided between the drive slider (2136) and the bottom of the second accommodation groove.

21. The drive structure of claim 19, wherein the drive claw (2130) is provided with a drive pin (2137), the drive slider (2136) is provided with a hole, and the drive pin (2137) is movably arranged through the hole of the drive slider (2136). The hole diameter is larger than the diameter of the drive pin (2137).

22. The drive structure of claim 1, wherein the operating component comprises a third operating piece (3210) that can be operated to move between the third position and the fourth position; When the third operating element (3210) is in the third position, the drive claw (3130) cooperates with the latch (3120), the latch (3120) is confined within the preset stroke, and the drive claw (3130) is in the first drive mode; When the third operating element (3210) is in the fourth position, the latch (3120) disengages from the drive claw (3130), and the drive claw (3130) is in the second drive mode.

23. The drive structure of claim 22, wherein the latch (3120) is arranged at the far end of the rack (3140), the latch (3120) is pivotly connected to the rack (3140) through a first rotating shaft (3121), and the first rotating shaft (3121) is provided with a first torsion spring (3122), The latch (3120) is elastically attached to the rack (3140) by the first torsion spring (3122).

24. The drive structure of claim 22, wherein the drive claw (3130) is arranged on the side of the trigger (3110) close to the rack (3140), and the trigger (3110) is rotatably connected to the drive claw (3130) through a second rotating shaft (3133) The second rotating shaft (3133) is provided with a second torsion spring (3134), and the relative position of the trigger (3110) and the driving claw (3130) is elastically restricted by the second torsion spring (3134).

25. The drive structure of claim 22, wherein a second limit slot (3135) is provided on the drive claw (3130), and when the latch (3120) is located in the second limit slot (3135), the latch (3120) cooperates with the drive claw (3130). When the latch (3120) is located outside the second limit slot (3135), the latch (3120) disengages from the drive claw (3130).

26. The drive structure of claim 22, wherein the third operating element (3210) comprises an operating end (3211) and a contact end (3212), and the operating end (3211) can be operated and moved, thereby driving the contact end (3212) to move up and down. The contact end (3212) is in contact with the drive claw (3130) for the purpose of driving the drive claw (3130) to move.

27. The drive structure of claim 26, wherein the third operating part (3210) comprises a linear motion part (3213) capable of moving up and down, and the linear motion part (3213) comprises an operating end (3211) protruding outward towards the handle housing (3190). And the contact end (3212) that protrudes into the handle housing (3190).

28. The drive structure of claim 27, wherein the third operating piece (3210) is arranged in the mounting slot inside the handle housing (3190); The drive structure includes a baffle (3215), which is used to limit the mounting position of the third operating element (3210).

29. The drive structure of claim 28, wherein the bottom of the third operating element (3210) is connected to the bottom of the mounting groove by a spring (3214) for the purpose of applying an elastic force to the third operating element (3210).

30. A surgical suture instrument, wherein it comprises a handle unit, an end actuator and a drive structure, and the operation of the handle unit regulates the working mode of the end actuator through the drive structure, and the drive structure comprises the drive structure of any one of claims 1-29.
